# EUROPEAN PATENT SPECIFICATION

(11) **EP 3 283 646 B1**
(45) Date of publication and mention of the grant of the patent: **04.09.2019**
(21) Application number: 16718440.7
(22) Date of filing: 13.04.2016
(51) Int. Cl.: C12Q 1/6806, C12Q 1/683, C12N 9/22, C12N 15/66

(54) **METHOD FOR ANALYSING NUCLEASE HYPERSENSITIVE SITES.**
VERFAHREN ZUR ANALYSE VON NUKLEASEHYPERSENSIBLEN STELLEN
PROCÉDÉ D'ANALYSE DES SITES HYPERSENSIBLES AUX NUCLÉASES

(30) Priority: 14.04.2015 GB 201506315
(43) Date of publication of application: 21.02.2018
(73) Proprietor: Belgian Volition SPRL, 5032 Isnes (BE)
(72) Inventor: ECCLESTON, Mark Edward, Ely Cambridgeshire CB6 3FL (GB); HERZOG, Marielle Chantal Andrée, 5000 Namur (BE)
(74) Representative: D Young & Co LLP
(86) International application number: PCT/GB2016/051031
(87) International publication number: WO 2016/166530

(56) References cited:
- GAETANO GARGIULO ET AL: "NA-Seq: A Discovery Tool for the Analysis of Chromatin Structure and Dynamics during Differentiation", DEVELOPMENTAL CELL, vol. 16, no. 3, 17 March 2009 (2009-03-17) , pages 466-481, XP055279824, US ISSN: 1534-5807, DOI: 10.1016/j.devcel.2009.02.002
- GREGORY E CRAWFORD ET AL: "DNase-chip: a high-resolution method to identify DNase I hypersensitive sites using tiled microarrays", NATURE METHODS, vol. 3, no. 7, 21 June 2006 (2006-06-21), pages 503-509, XP055279821, GB ISSN: 1548-7091, DOI: 10.1038/nmeth888
- L. SONG ET AL: "DNase-seq: A High-Resolution Technique for Mapping Active Gene Regulatory Elements across the Genome from Mammalian Cells", COLD SPRING HARBOR PROTOCOLS, vol. 2010, no. 2, 1 February 2010 (2010-02-01), XP055279823, DOI: 10.1101/pdb.prot5384
- ALAN P. BOYLE ET AL: "High-Resolution Mapping and Characterization of Open Chromatin across the Genome", CELL, vol. 132, no. 2, 1 January 2008 (2008-01-01), pages 311-322, XP055247811, US ISSN: 0092-8674, DOI: 10.1016/j.cell.2007.12.014

## Description

### FIELD OF THE INVENTION

The present invention relates to a high throughput, high resolution technique for mapping multiple hypersensitive sites (e.g. the integrated analysis of genes, of regulatory elements and chromatin architecture) across a mammalian (preferably human) genome.

### BACKGROUND

The genome of a particular species consists of a unique sequence of DNA with each cell carrying identical copies of this genetic code. Selective activation of specific regulatory regions of the DNA provides a mechanism for cellular differentiation. These non-sequence driven changes exert epigenetic control of gene expression and allow much greater cell diversity within an organism, for example the 200 or so specific (yet genetically identical) cell types that make up a human.

A key factor in this process is the accessibility, and cooperative binding, of transcriptional regulators to defined sequences of DNA within chromatin, a condensed DNA - histone protein structure used to package DNA within the confines of the cell nucleus. Thus, at a basic level, the chromatin structure regulates gene expression and ensures genes encoding for liver specific proteins are active in the liver and genes encoding proteins specific to other tissues such as nerve cells or muscle cells are not active.

Misregulation of epigenetic signaling is a factor in many diseases. This is particularly true of cancer where activation of oncogenes and inactivation of tumour suppressor genes is key. The classical "two hit" theory of oncogenesis holds that deactivating mutations in both alleles of a cancer suppressor gene are required for its tumour suppression effect to be lost.

It is now appreciated that such inactivation can occur equally by epigenetic switching of tumour suppressor genes (sometimes called the "third hit"). Similarly, oncogenes may be activated epigenetically. Accumulation of such changes leads to disease development and progression. Such epigenetic changes play a role in all cancers and epigenetic drugs, aimed at inhibiting such changes, are in clinical use.

Within chromatin the DNA is structured on several levels. Initially it is coiled around histone proteins to create nucleosomal DNA-protein complexes. Subsequent coiling of these nucleosomal complexes into solenoid and higher order structures increases the packaging density further. In fact, the majority of DNA is within closed, condensed heterochomatin domains, which are inaccessible to cell transcription machinery. However, regulatory regions of DNA are largely devoid of nucleosomes and adopt a more open, euchromatin, structure, allowing access to trans regulatory molecules. Each nucleosome consists of around 150 base pairs of DNA and the open regulatory regions are typically up to a thousand base pairs in length.

The pattern of open elements across the genome is characteristic of a cell type or state. These open regions display heightened sensitivity (typically 100x) to nuclease activity compared to condensed heterochomatin. Hence these Nuclease Accessible Site (NAS) are also referred to as Hypersensitive (HS) sites.

Around 2.9 million HSs have been identified across the human genome in an extensive evaluation of 125 different healthy and diseased cell and tissue types (Thurman et. al. Nature; 2012,doi:10.1038/nature11232*).* Around a third are unique to a particular cell type. Two thirds are found in more than one cell type but less than 0.13% are found in all cell types. 5% of HSs are found within 2.5Kb of a transcription start site, including a strong correlation between HSs location and Transcription Start Sites for micro RNA (a major class of regulatory molecules). The remaining 95% are distributed relatively evenly throughout the intronic and intergenic regions although there is some enrichment of HSs at Long Terminal Repeat elements associated with retroviral enhancer structures. It is largely these distally positioned HSs that are cell specific. The number of HSs in any single cell type is much lower, typically around 300000, which gives a mean length between adjacent HS of 10000 base pairs. Thus, nucleic acid fragments produced by selective nuclease digestion will be, on average, 10000 base pairs long which has important consequences for methods used to analyse chromatin structure in terms of HSs as detailed below.

HSs have been investigated over the past 30 years at an individual gene level by Southern blotting. This method is not suitable for genome wide screening given the large number of potential sites. Briefly these methods involve isolation of a short length of chromatin usually covering a single gene. The chromatin is exposed to a nuclease which preferentially cuts the chromatin at exposed HS regions. The DNA fragments produced are then extracted from the chromatin and separated electrophoretically according to size, transferred by blot and hybridised with a radiolabelled recombinant DNA probe for analysis to determine the location of HS regions in the gene. This is not suitable as a routine method for genome wide comparison of multiple cell types.

More recently, genome wide methods have been described by Crawford and Minucci.
In the method described by Crawford (e.g. Crawford et al. Genome; Methods; 2006; doi/10.1101/gr.4074106; Crawford et al. Nat. Methods; 2006; doi:10.1038/NMETH888; Boyle et. al. Cell; 2008; DOI 10.1016/j.cell.2007.12.014 and Song & Crawford Cold Spring Harb. Protoc; 2010; doi:10.1101/pdb.prot5384) whole chromatin, extracted from cell nuclei, is first treated with a non-sequence specific DNAse to cut Hypersensitive sites. The fragments are embedded in low melting agarose, treated over night with surfactant and washed exhaustively to remove bound protein followed by a buffer exchange. The DNA is then blunt ended and a biotinylated adaptor, containing a recognition site for a second restriction enzyme, ligated to the blunt ends. A second, sequence specific nuclease, is used to cut 20 base pairs distally to the specific sequence introduced within the first adapter generating fragments with a uniform size and bearing a 2 base pair degenerate overhang. At this point the fragments are isolated from the gel on streptavidin beads and a second set of adapters ligated to the sticky end. Sequence ready (Illumina) libraries of fragments were produced by PCR amplification from the beads and purification by electrophoresis to remove non-ligated adaptors.

Attempts to reduce the background noise, typically seen during non specific DNAse approaches, by utilizing sequence specific restriction enzymes have been used e.g. Gargiulo & Minucci; Cell Press. Developmental Cell; 2009; DOI 10.1016/j.devcel.2009.02.002. In this approach a sequence specific nuclease, or combination of multiple sequence specific nucleases, is used to generate primary cuts with sequence specific sticky ends at the Hypersensitive sites of intact chromatin. The resulting fragments are embedded in low melting agarose gel and treated overnight with Proteinase K followed by washing to remove protein components of the chromatin. The resulting high molecular weight fragments are subsequently treated with RNAse to digest RNA and treated with an additional sequence specific nuclease, SAU3AI, to reduce the fragment size and introduce a second distinct sequence specific sticky end. These fragments are electrophoresed directly from the agarose gels into 0.8% agarose gel and subsequently purified using a commercial gel extraction kit (Qiagen). Biotinylated and one non-biotinylated sequencing adapter pairs are ligated unidirectionally by virtue of sticky ends complementary to the sticky ends introduced during the digestion phases. Sequence ready (454) libraries can then be prepared by enrichment and PCR amplification of biotinylated fragments on streptavidin beads.

The inventors have found the prior art methods to have many limitations.

The present invention seeks to provide an effective, high-throughput, low-cost method for mapping multiple hypersensitive sites (e.g. the integrated analysis of genes, of regulatory elements and chromatin architecture) across a mammalian (preferably human) genome.

### SUMMARY OF THE INVENTION

According to a broad aspect the present disclosure provides a method for mapping multiple hypersensitive sites across a mammalian (preferably human) genome comprising:
a. fragmenting a nucleic acid sample comprising chromatin (e.g. genomic DNA) at multiple hypersensitive sites by treating the nucleic acid sample comprising chromatin with a first sequence specific restriction enzyme which restriction enzyme leaves a sticky end,
b. ligating an adapter oligonucleotide onto the sticky end produced by the first sequence specific restriction enzyme, which adaptor oligonucleotide contains a single stranded region which is complementary to the sticky end produced by the first sequence specific restriction enzyme, and which adaptor oligonucleotide contains a recognition site (e.g. target DNA sequence) for a second restriction enzyme,
c. treating the fragments with said second sequence specific restriction enzyme which second sequence specific restriction enzyme cuts at a position at a defined number of bases distal to said recognition site (e.g. wherein the known distance is one which is between 16 and 50bp), to provide fragments having an identical sequence at one end thereof and being all the same size;
   wherein at least steps b. and c. are conducted in an aqueous medium.

This method may further comprise analyzing the fragments obtained in step c.

According to one aspect the present invention provides a method for analysing nuclease hypersensitive sites which method comprises:
i) cleaving a nucleic acid sample comprising chromatin at multiple nuclease hypersensitive sites with a first sequence specific restriction enzyme to introduces a staggered cut and leave a single chain 3' or 5' overhang in a double stranded DNA;
ii) optionally isolating substantially free DNA from the digested nucleic acid sample or removing the protein and RNA components from the digested nucleic acid sample to leave substantially free DNA;
iii) ligating an adapter oligonucleotide onto the overhang produced by the first sequence specific restriction enzyme in aqueous solution, which adaptor oligonucleotide contains a single stranded region which is complementary to the overhang produced by the first sequence specific restriction enzyme, and which adaptor oligonucleotide contains a recognition site (e.g. target DNA sequence) for a second restriction enzyme;
iv) treating the ligated DNA sequence with a second restriction enzyme wherein said second restriction enzyme is specific to said recognition site introduced within said adaptor oligonucleotide, wherein said second restriction enzyme cuts at a position at a defined number of bases distal to said recognition site and introduces a staggered cut, leaving a single chain 3' or 5' overhang in the double stranded DNA;
v) optionally amplifying the DNA fragments;
vi) analysing the DNA fragments formed in iv) or v) from a plurality of sequences (such as a plurality of genes)
wherein at least steps iii) and iv) of the method are conducted in an aqueous medium.

Suitably in some embodiments of the present invention, step ii) of the method is also conducted in an aqueous medium.

Suitably in some embodiments the method of the present invention may comprise after step (iv) a step of ligating a second oligonucleotide adaptor, which second oligonucleotide adaptor has a single stranded region which is complementary to the overhang produced by the second sequence specific restriction enzyme, to the DNA fragments.

In one embodiment the DNA fragments obtained in step iv) or following the ligation of a second oligonucleotide adaptor taught above may be amplified.

In a further aspect the present invention provides a kit for the preparation of hypersensitive site libraries which kit comprises:
i) a first sequence specific restriction enzyme capable of introducing a staggered cut and leaving a single chain 3' or 5' overhang in a double stranded DNA of a nucleic acid sample;
ii) an adapter oligonucleotide containing a single stranded region which is complementary to the overhang produced by the first sequence specific restriction enzyme, and which adaptor oligonucleotide contains a recognition site (e.g. target DNA sequence) for a second restriction enzyme;
iii) a second restriction enzyme which is specific to said recognition site of said adaptor oligonucleotide, wherein said second restriction enzyme cuts at a position at a defined number of bases distal to said recognition site and introduces a staggered cut, leaving a single chain 3' or 5' overhang in the double stranded DNA.

The kit may further comprise a second oligonucleotide adaptor, e.g. a set of degenerate adaptors, which second oligonucleotide adaptor(s) has a single stranded region which is complementary to the overhang produced by the second sequence specific restriction enzyme, to the DNA fragments.

### BRIEF DESCRIPTION OF THE DRAWINGS

Embodiments of the invention will now be described, by way of example only, with reference to accompanying drawings, in which:
Figure 1 shows a rapid throughput nuclease hypersensitive site mapping workflow
Figure 2 shows optimisation of nuclei digestion. (A) Agarose gel of DNA from nuclei digested with NlaIII (1U/µl) with incubation time (B) Agarose gel analysis of DNA from nuclei digested with increasing NlaIII concentration at 5 min fixed incubation time. L1= 1kb extension ladder, L2= 1kb Plus ladder
Figure 3 shows agarose gel analysis of DNA from nuclei (Jurkat cells) digested with Nlalll enzyme at 0.1U for varying incubation periods. 10µl (≈ 1µg) of each sample was analysed on 1% gel agarose. L1= 1Kb extension ladder, L2= 1Kb Plus Ladder.
Figure 4 shows agarose gel analysis of DNA from nuclei (Jurkat cells) digested with different concentration of NlaIII enzyme for 30min. 10µl (≈ 1µg) of each sample was analysed on 1% gel agarose. L1= 1Kb extension ladder, L2= 1Kb Plus Ladder
Figure 5 shows a garose gel analysis of DNA from nuclei (Jurkat cells) digested with 0U, 0,05 or 0,07U of NlaIII enzyme for 10 min. incubation. 10µl (≈ 1µg) of each sample was analysed on 0,7% gel agarose.
Figure 6 shows agarose gel analysis of digested DNA from 3.10⁶ nuclei with 0.5U Nlalll at 37°C for 1 hour followed by by followed by RNAse and Proteinase K treatment. 10µl (∼ 1µg) was analysed on 0.7% gel agarose. Bands at low bp indicative of mono-,di-, tri- and oligonucleosome formation.
Figure 7 shows agarose gel analysis of DNA from Jurkat cell nuclei digested with Nlalll (low and high concentration) at 37°C. DNA was purified or not using Wizard SV gel and PCR clean up kit from Promega.10µl of each sample was analysed on 0,7% agarose gel. E1: first elution, E2: second elution
Figure 8 shows agarose gel analysis of DNA from Jurkat cell nuclei digested with 0.1U NlaIII at 37°C for 10 minutes following RNAse and Proteinase K treatment. 10µl (∼ 1µg) was analysed on 0.7% gel agarose and purified (1mL) by wizard SV gel system (W), RNAse treatment only and purified (200µL) by Akonni TruTip® system (A1), no RNAse or Proteinase K treatment and purified (200µL) by Akonni TruTip® system (A2). (Akonni Biosystems 400 Sagner Ave., Suite 300 Frederick, MD 21701, US)

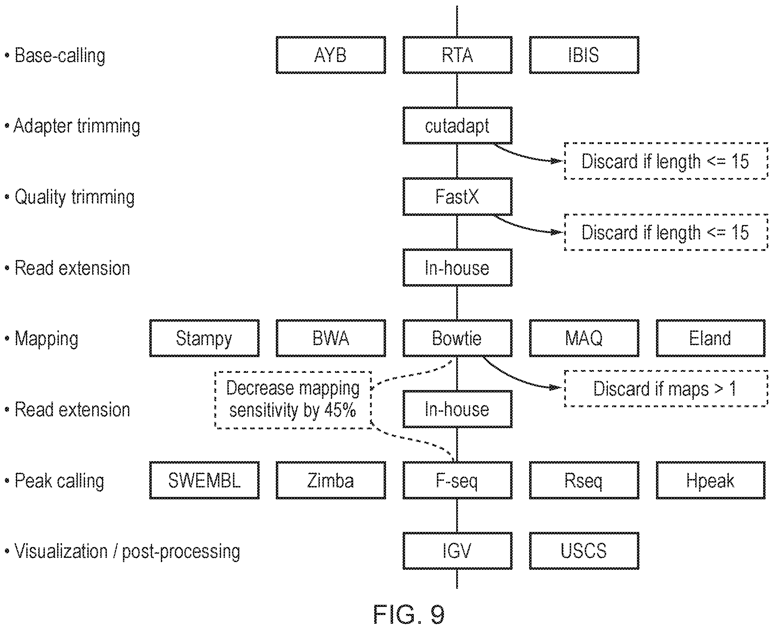

Figure 9 shows bioinformatics pipeline for analysis of Next Generation Sequencing Data derived from hypersensitive site libraries prepared by the present invention - Hyper-Seq™ data, Primary analysis was carried out using the Illumina Real Time Analysis (RTA) software package to perform base calling and quality scoring. Files were pre-processed using the Cutadapt (source code available from MIT; http://code.google.com/p/cutadapt/.) was used to remove adapter sequences (Adapter Trimming) and the FastX-Toolkit (hannonlab.cshl.edu) to remove reads with low quality scores (Quality Trimming). An in house algorithm developed by /Biomedicum-Genomics (Biomedicum Genomics Oy, Haartmaninkatu 8, FI-00290 Helsinki, Finland was used to extend the reads before mapping to the human genome using the ultrafast memory-efficient short read aligner software package - Bowtie (available to download from sourceforge.net). alternative packages including Stampy, BWA, MAQ and Eland are available. Reads mapping to more than one unique site within the reference human genome were discarded. Peak calling was performed using F-Seq, a density estimator for high throughput sequencing tags developed by the Terry Furey Lab (University of North Carolina - http://fureylab.web.unc.edu/software/fseq/). Alternative software packages include SWEMBL, Zimba, RSeq and HPeak. Fially, the data was visualised using the Integrated Genomics Viewer, an high performance viualisation tool for interactive exploration of large, integrated genomic data sets developed at the Broad Institute (www.broadinstitute.org/igv/)
Figure 10 shows agarose gel of off bead PCR product from biotinylated hypersensitive site libraries from Jurkat cells with secondary ligations times of 1hr (T1), 2 hrs (T2) and 4 hrs (T3) indicating ligation is essentially complete after one hour.
Figure 11 shows agarose gel of off bead PCR product from biotinylated hypersensitive site libraries from PBMC, oestrogen stimulated and non-stimulated MCF7 and Jurkat cells. The band at 86bp corresponds to the sequence ready linker-insert combination. M:Ultra low range DNA ladder.
Figure 12 shows quality Scores across all base pairs (Illumina 1.5 encoding) for Jurkat, PBMC and first serial sample of PBMC cells (Examples 1-3),
Figure 13 shows enrichment of Hypersensitive site peaks mapping to the EnsEMBL TSS database. 10051 peaks mapped from Jurkat cells are within the 1000bp from known transcription start sites.
Figure 14 shows heat map showing Hypersensitive site distribution from Jurkat, PBMC and first serial PBMC samples across chromosomes 1-8.
Figure 15 shows unrooted phylogenetic tree showing relationship between Jurkat, PBMC and first serial PBMC samples.
Figure 16 shows a common Nuclease Hypersensitive site appearing on human primary Peripheral Blood Mononucleocyte cells and the Jurkat cell line and a Jurkat specific Nuclease Hypersensitive site within chromosome 2 in a screenshot of HSs from the Ensembl Genome Browser viewer.
Figure 17 shows an unrooted phylogenetic tree showing Nuclease Hypersensitive site correlation between Jurkat technical and biological repeats as well as overdigested samples, PBMC technical repeats and second and third serial PBMC samples.
Figure 18 shows three common Nuclease Hypersensitive Sites appearing in oestrogen stimulated and non-stimulated MCF7 cells and one differential Nuclease Hypersensitive Sensitive appearing on chromosome 7 in a screenshot of HSs from the Ensembl Genome Browser viewer.
Figure 19 shows differential Nuclease Hypersensitive Sites appearing within an intron of the PTK2 gene on chromosome 8 in non-oestrogen stimulated MCF7 cells but not in oestrogen stimulated MCF7 cells 7 in a screenshot of HSs from the Ensembl Genome Browser viewer.

### DETAILED DESCRIPTION

The ability to usefully analyse nuclease Hypersensitive sites depends on the ability to differentiate genuine Nuclease Hypersensitive sites from random breaks introduced as a result of sample processing. Traditional Genome Wide Nuclease Hypersensitive site profiling approaches have attempted to overcome this limitation by stabilisation, and subsequent processing, of post nuclease treated chromatin fragments in low melting gel systems. This substantially extends the processing time due to the slow reaction kinetics in a gel compared to standard aqueous solution buffers.

A seminal finding of the present invention is that by employing the methods described herein, Genome Wide Nuclease Hypersensitive Site Mapping can be performed without stabilisation of process intermediates, e.g. in gels, thus significantly simplifying the process of isolation of DNA sequences from Nuclease HyperSensitive sites.

For the first time the present inventors have shown that libraries of DNA sequences from Nuclease Hypersensitive Sites can be isolated and/or detected and/or analysed using an aqueous medium based protocol that significantly reduces sample processing time and is suited for high throughput sample processing.

The present invention is predicated upon the surprising finding that tagging Nuclease Hypersensitive sites with an adaptor at a defined position within a Nuclease Hypersensitive site, wherein the adapter contains a target sequence for a second restriction enzyme that cuts at a defined distance distal to that sequence, allows isolation and/or detection and/or analysis of nucleic acid sequences of defined length from genuine Nuclease Hypersensitive sites rather than random breaks introduced during sample processing.

The present invention has reduced background signal, reduced loss signal and/or a reduced processing time compared with conventional methods.

In particular, a rapid through-put method is achievable by having a rapid library preparation, which is achievable by using an aqueous medium during these stages, which aqueous medium is not hindered by the reduced reaction kinetics within gels.

Conventionally gels have been used during the various enzymatic processing steps to prepare HSs libraries which makes the methods slow and laborious.

The present disclosure relates to a rapid, high throughput method of identifying nuclease hypersensitive sites (preferably on a genome-wide scale).

The present disclosure provides methods for the identification, isolation and characterisation of collections of DNA sequences (fragments) of defined length from nuclease hypersensitive sites in a high throughput manor without requiring prior knowledge of the location or function of said DNA sequences within the genome.

The inventors have found a number of limitations in genome wide methods known in the art which the present invention seeks to address, such as:
i) The long DNA fragments created by the first mild digestion are highly prone to random breaks in solution. This can lead to high background noise associated with random breaks. In addition or alternatively loss of amplifiable sequences can occur due to random fragment cleavage since random breaks will not have sticky ends required for annealing adaptors for amplification and thus will not be detected resulting in loss of signal. Thus in such situations one or more subsequent processing steps are performed in a low melting agarose gel in order to reduce this problem. The gel matrix limits the movement of the DNA fragments to a large extent and protects their integrity. In most gels DNA fragments may remain stationary for many days (for example in a DNA band produced by electrophoresis in a gel) unless an external force such as an electric potential is applied to force their movement. The use of gels minimises random DNA breakage but limits and slows the methods in two important ways; firstly it slows the kinetics of the reactions used and hence slows the entire process, and secondly it renders automation of the process impractical.
ii) The positions of restriction enzyme sites are not evenly spaced in the genome and the DNA fragment libraries produced may be therefore highly heterogeneous in size. In addition, analysis of the libraries involves PCR amplification and sequencing. However, PCR amplification efficiency is DNA size dependent and the amplified libraries produced will inevitably be skewed towards smaller fragments. Next Generation sequencing frequently employs a sizing step to select a particular range of fragments for sequencing. Any fragments outside the selected range will not be sequenced resulting in a loss of information.

In the present invention we provide a method for analysing nuclease hypersensitive sites which method comprises:
i) cleaving a nucleic acid sample comprising chromatin at multiple nuclease hypersensitive sites with a first sequence specific restriction enzyme to introduces a staggered cut and leave a single chain 3' or 5' overhang in a double stranded DNA;
ii) optionally isolating substantially free DNA from the digested nucleic acid sample or removing the protein and RNA components from the digested nucleic acid sample to leave substantially free DNA;
iii) ligating an adapter oligonucleotide onto the overhang produced by the first sequence specific restriction enzyme in aqueous solution, which adaptor oligonucleotide contains a single stranded region which is complementary to the overhang produced by the first sequence specific restriction enzyme, and which adaptor oligonucleotide contains a recognition site (e.g. target DNA sequence) for a second restriction enzyme;
iv) treating the ligated DNA sequence with a second restriction enzyme wherein said second restriction enzyme is specific to said recognition site introduced within said adaptor oligonucleotide, wherein said second restriction enzyme cuts at a position at a defined number of bases distal to said recognition site and introduces a staggered cut, leaving a single chain 3' or 5' overhang in the double stranded DNA;
v) optionally amplifying the DNA fragments;
vi) analysing the DNA fragments formed in iv) or v) from a plurality of sequences (such as a plurality of genes).
wherein at least steps iii) and iv) of the method are conducted in an aqueous medium.

Suitably in some embodiments of the present invention, step ii) of the method is also conducted in an aqueous medium.

Suitably in some embodiments the method of the present invention may comprise after step (iv) a step of ligating a second oligonucleotide adaptor, which second oligonucleotide adaptor has a single stranded region which is complementary to the overhang produced by the second sequence specific restriction enzyme, to the DNA fragments.

The term "substantially free DNA" as used herein means a DNA that has been isolated from the protein components of a nucleic acid sample comprising chromatin.

In one embodiment preferably the first sequence specific restriction enzyme is a restriction enzyme which targets a specific, known sequence within nuclease hypersensitive regions.

A nuclease which introduces a staggered cut in accordance with the present invention is preferably one that cuts off centre from the original recognition site e.g. within the nuclease hypersensitive sites.

The single chain 3' or 5' overhang in the double stranded DNA which is produced by the sequence specific nuclease is also known as a sticky end. These terms are used interchangeably herein.

In one embodiment the method of the present invention may be used for determining the presence of and/or analyzing and/or mapping Nuclease Hypersensitive Sites on a global and genome wide scale.

In an alternative embodiment the method of the present invention may be used for determining the presence of and/or analyzing and/or mapping Nuclease Hypersensitive Sites on a chromosome.

In another embodiment mutliple restriction enzymes may be used concurrently in the method of the present invention to introduce targeted cuts into DNA sequences present in HSs.

In one embodiment the nucleic acid sample comprising chromatin for use in the present invention is genomic DNA.

The restriction enzymes used in accordance with the present invention may be engineered restriction enzymes with improved target specificity, reduced off target (star) activity and optimized performance over a wide range of digestion conditions. Examples include the New England Biolab High-Fidelity (HT®) range of restriction enzymes (Kamps-Hughes et.al. Nucleic Acids Research; 2013; doi: 10.1093/nar/gkt257) available from New England Biolabs Inc. (240 Country Road, Ipswich, MA, US)

in some embodiments, Zinc Finger nuclease enzymes may be used as the first or second sequence specific restriction enzyme in the method of the present invention to introduce targeted cuts into DNA sequences present in HSs.

There are many advantages associated with the present invention. By way of example only the method of the present invention provides for rapid analysis of nuclease hypersensitive sites on a global and genome-wide scale. "Rapid analysis" as used herein means sample to library preparation in 48 hours or less. For instance cleaving a nucleic acid sample comprising chromatin at multiple nuclease hypersensitive sites with a first sequence specific restriction enzyme to introduces a staggered cut and leave a single chain 3' or 5' overhang in the double stranded DNA can typically be undertaken in less than 1 hour; ligating an adapter oligonucleotide onto the overhang produced by the first sequence specific restriction enzyme in aqueous solution, which adaptor oligonucleotide contains a single stranded region which is complementary to the overhang produced by the first sequence specific restriction enzyme, and which adaptor oligonucleotide contains a recognition site (e.g. target DNA sequence) for a second restriction enzyme takes approximately 1 hour; and/or treating the ligated DNA sequence with a second restriction enzyme wherein said second restriction enzyme is specific to said recognition site introduced within said adaptor oligonucleotide, wherein said second restriction enzyme cuts at a position at a defined number of bases distal to said recognition site and introduces a staggered cut, leaving a single chain 3' or 5' overhang in the double stranded DNA additionally takes approximately 1 hour.

This contrasts sharply with prior art methods where some of these stages take more than 8h, even 12 hours to complete. Hence with the present invention it is possible to speed the process up and reduce the analysis time to less than 2 days, preferably less than 1 day.

The method of the present invention is suitable for automation. In particular, the analysis and/or determining and/or mapping of nuclease hypersensitive sites on a global and genome-wide scale using the method according to the present invention means it is amenable to high through-put automation using for example liquid handling robots. This can be achieved because the major steps (e.g the chemistry steps) of the method are carried out in an aqueous medium (e.g. rather than a gel phase).

Notably the chemistry steps are those where gel kinetics slow the reaction and include for example at least steps iii) and iv) of the method of the present invention.

In one embodiment step ii) is also considered a chemistry step.

Notably purification steps for example to remove non-ligated primary and secondary adapter oligonucleotides, can be still carried out in a gel as these are generally rapid as they are not slowed by gel kinetics. However, in many instances alternatives exist to the purification and thus even the purification steps may be carried out without the use of gels.

The method of the present invention is thus amenable for high throughput determination and/or mapping and/or analysis of nuclease hypersensitive sites on a global and genome-wide scale.

The term "high throughput" as used herein means parallel processing of multiple samples

A method for producing DNA sequences (fragments) of defined length from defined regions within nuclease hypersensitive sites is also taught herein. These defined regions are determined by the first sequence specific restriction enzyme.

The method of the present invention produces DNA sequences (fragments) of a defined length from defined regions within nuclease hypersensitive sites that obviates the need to protect the DNA sequences from random fragmentation during processing. This can lead to significant advantages over prior art methods.

The method of the present invention is carried out in an aqueous medium. In particular, the steps in the method for producing DNA sequences of defined length from defined regions within nuclease hypersensitive sites are carried out in an aqueous medium.

In one preferred embodiment, the method of the present invention comprises use of the polymerase chain reaction in combination with adapter oligonucleotides specifically ligated to each end of the defined length DNA sequence (fragment) to amplify a library of the DNA sequences (fragments).

In an alternative embodiment, the DNA sequence(s) (fragments) are sequenced. By way of example only Oxford Nanopore technology enables direct sequencing of the fragments without the need for amplification, e.g. PCR amplification (Timp et. Al.Biophysical Journal; 2012; DOI: http://dx.doi.org/10.1016/j.bpj.2012.04.009).

The method of the present invention preferably comprises analyzing a library of DNA sequences (fragments) obtained by the present method from multiple nuclease hypersensitive sites by Next Generation Sequencing. Library sequencing is enabled by virtue of sequencing primer target sequences and an adapter sequence (which complement the Illumina HiSeq platform) included within the oligonucleotides annealed to the sticky ends of the fragments. These linkers are modified from the DNAse-seq method (Song & Crawford Cold Spring Harb. Protoc; 2010; doi:10.1101/pdb.prot5384.). Sequencing comprises:
i) binding single stranded fragments from a hypersensitive site library to the inside surface of an Illumina flow cell channel via an adapter.
ii) cluster generation via bridge amplification using Illumina PCR primers.
iii) sequencing by synthesis on an Illumina GAIIx or HiSeq instrument using a 36 cycle, single read protocol
iv) base calling and error correction
v) removal of redundant reads (those not containing the restriction target sequence)
vi) alignment of the reads to the reference human genome
vii) bioinformatics analysis (detailed in Figure 9) and identification of enriched sequences

in one aspect, the method of the present invention may comprise analyzing a library of DNA sequences (fragments) obtained by the present method from multiple nuclease hypersensitive sites by microarray. Library analysis follows a modification of the method described by Crawford (Crawford et. Al. Nat. Methods; 2006; doi:10.1038/NMETH888) comprising:
i) incorporation of a Cy3-dUTP label into the DNA fragments during the PCR amplification phase.
ii) generating a second library incorporating Cy5-dUTP wherein the nucleic acid sample is first treated with a protease to remove all protein and thus generate substantially free DNA in which all restriction enzyme target sites are equally accessible.
iii) combining the two samples with a blocking buffer (tRNA, Cot-1 DNA, poly(A)⁺RNA and poly(T)⁺RNA followed by ethanol precipitation
iv) resuspending the pellet in an aqueous hybridization buffer (50%formamide, 10%SSC and 0.4%SDS) and hybridizing the samples for 20 > hrs to a Nimblegen ENCODE tiled array which consists of approximately 385,000 50-mer oligos spaced approximately every 38bp of the non repetitive fraction of the human genome (Nimble-gen).
v) washing the slides and scanning (Agilent array reader)
vi) normalizing signals using Niblescan software (NimbleGen) and applying a ² test on sliding 500-bp windows to identify genomic regions with higher than expected numbers of oligos in the top 5% of the log-ratio distribution (P<0.001) indicative of hypersensitive sites

In another aspect, the method of the present invention may include using the polymerase chain reaction in combination with an adapter oligonucleotide ligated to the primary HSs cut site and a set of short arbitrary primers to known genomic regions to generate a series of one-dimensional representations of nuclease hypersensitive sites by electrophoresis. (See Giresi and Lieb Nature Methods; 2006; doi:10.1038/nmeth0706-501 for a low plex version of this approach)

According to another aspect of the present invention there is provided a kit for preparation of DNA sequences of defined length from defined regions within nuclease hypersensitive sites comprising:
i) an enzyme capable of introducing a staggered cut and leaving a single chain 3' or 5' overhang in the double stranded DNA of a nucleic acid sample;
ii) an adapter oligonucleotide containing a single stranded region which is complementary to the overhang produced by the first sequence specific restriction enzyme, and which adaptor oligonucleotide contains a recognition site (e.g. target DNA sequence) for a second restriction enzyme; and
iii) a second restriction enzyme wherein said second restriction enzyme is specific to said recognition site introduced within said adaptor oligonucleotide, wherein said second restriction enzyme cuts at a position at a defined number of bases distal to said recognition site and introduces a staggered cut, leaving a single chain 3' or 5'overhang in the double stranded DNA;
iv) optionally a second oligonucleotide adaptor, which second oligonucleotide adaptor has a single stranded region which is complementary to the overhang produced by the second sequence specific restriction enzyme, to the DNA fragments.

In one embodiment preferably the second oligonucleotide adaptor is a set of degenerate adaptors.

The term "set of degenerate adaptors" means more that one adaptor wherein each adaptor has a different single stranded region which is complementary to the non-specific two base overhand sticky ends produced by the second sequence specific restriction enzyme. In other words the set of degenerate adaptors provides a set of adaptors having single stranded regions complement to each possible combination of two-base overhang produced by the second sequence specific restriction enzyme. It will be clear to one skilled in the art that the cleavage products for the second sequence specific restriction enzyme (e.g. the Mme1 enzyme) will have non-specific 2 base overhang sticky ends i.e. sequences containing each possible combination of 2 base overhang.

In some embodiments, the second oligonucleotide adapter(s) may contain a primer sequence complementary to the first primer sequence introduced via the primary adapter.

The methods of the present invention may be suitable for any organism, in particular any eukaryotic organisms. In one preferred embodiment the organism is a mammal. In one preferred embodiment the organism is a human.

A further advantage of the methods of the present invention is that it does not require prior knowledge of the location or function of said DNA sequences (fragments) within the genome.

In some embodiments, the method of the present invention may include a pre-step for the isolation and stabilisation of cell nuclei.

In some embodiments the pre-step for the isolation and stabilisation of cell nuclei may not be necessary. By way of example only the first digestion (e.g. the fragmenting of the genomic DNA or the cleavage of the nucleic acid sample with the first sequence specific nuclease) could be carried out intracellularly followed by extraction of the chromatin fragments from the cell.

The cells from which the DNA may be obtained, include immortalised cells from in-vitro tissue culture; primary cells from in-vitro culture, cells from ex-vivo tissue culture, 3-dimensional cell cultures, blood cells, for example cells isolated from a buffy layer following whole blood sampling, tissue samples, for example clinical biopsies, biopsies from an organism wherein the biopsy is obtained from a diseased tissue, cells isolated from a host organism, wherein the host has a specific disease or stem cells or primary cells that have been partially or completely reverted back to stem cell status i.e. induced pluripotent stem cells.

In some embodiments the method of the present invention may further comprise extracting intact chromatin from cell nuclei.

Where the method comprises a step of extracting intact chromatin from cell nuclei, this may be achieved by contacting the isolated nuclei with an extraction buffer to remove the nuclear membrane to produce cell free, intact chromatin.

The first sequence specific restriction enzyme may be naturally occurring. Alternatively the first sequence specific restriction enzyme may be a synthetic or engineered restriction enzyme such as a Zinc Finger Nuclease provided that the enzyme introduces a cut with an overhang or sticky end capable of distinguishing between a targeted cut and a random break within the nuclease hypersensitive sites.

In one embodiment the fragmented nucleic acid or genomic DNA (e.g. free nucleic acid or DNA fragmented by exposure to the first sequence specific restriction enzyme) may be isolated from the chromatin.

The present method is highly advantageous in this regard as the fragmented chromatin (e.g. post fragmentation with the first sequence specific restriction enzyme) may be treated with RNAse and protease in aqueous media to give substantially free DNA which is subsequently processed further to provide libraries of DNA of suitable size for further analysis.

Importantly, due to the presence of the specific sticky end post-fragmentation with the first sequence specific restriction enzyme, random chemical or mechanical fragmentation of the DNA during these processing steps is well tolerated in the method of the present invention. In other words, non-specific fragmentation of the DNA is not recognised in subsequent processing steps, which is a significant source of background noise when using non-sequence specific cleavage such as chemical, mechanical, DNAse or MNase based primary cleavage. Beneficially, sticky end ligations require less reaction time than blunt ended ligations.

The DNA sequences (fragments) may be isolated using standard methods including phenol//chloroform extraction followed by precipitation and dissolution of the DNA or by binding DNA to a matrix followed by washing and elution.

As noted above an adapter is introduced to the DNA fragments isolated from a nuclease hypersensitive sites region post fragmentation with the first sequence specific restriction enzyme by virtue of its sticky end.

An adapter (e.g. a first adaptor), specific to the sticky end of the DNA fragments is ligated to the sticky end of the isolated DNA fragments. In one embodiment, the adapter preferably contains a primer designed for PCR amplification.

More preferably the first adapter also contains an affinity tag for isolation of the ligated fragments. The affinity tag can be biotin, thus allowing isolation of the adapter ligated fragments using a streptavidin or avidin matrix.

It is essential in any event that the first adapter contains a recognition site for a second restriction enzyme. Importantly, fragments containing random breaks or breaks introduced by non-specific activity of the restriction enzyme, also known as star activity, are not ligated and are therefore not co-purified substantially reducing the potential for background in subsequent analysis of the fragments.

Optionally the (first) adapter can also contain a sequencing primer.

The (first) adapter may also contain a multiplex indexing tag.

An essential aspect of the present invention is that the method produced uniform sized DNA for analysis.

To achieve this, a second (sequence specific) restriction enzyme digest is carried out on the adapter ligated DNA fragments wherein the second restriction enzyme is specific to the recognition site introduced within the first adaptor. The second restriction enzyme is selected based on its capability to cut at a position at a defined number of base pairs distal to the recognition site introduced on the first fragment. Preferably the size of the resulting doubly digested DNA fragments is sufficient to allow them to be uniquely identified relative to the genome of host cells from which the DNA fragments were originally isolated.

In some embodiments, the second restriction enzyme may introduce a staggered cut, e.g. to leave a second sticky end.

A second adaptor, complementary to the second sticky end, may then ligated to the DNA sequences. Preferably the second adapter when present contains a primer complementary to that introduced on the first adapter allowing the fragments to be amplified if necessary prior to analysis.

Importantly, tagging genuine Nuclease Hypersensitive sites with a (first) adaptor containing a recognition site for a second sequence specific restriction enzyme site overcomes potential loss of amplifiable sequences that can result from approaches using secondary restriction enzymes targeting natural sites within the isolated primary DNA sequences. Non-specific cleavage between two complementary restriction enzyme target sites would leave fragments with one non-ligateable end thus not be amplified and/or sequenced.

Restriction Enzymes are also known as restriction endonucleases or nucleases (these terms may be used interchangeably herein). Restriction enzymes allow sequence specific DNA cleavage within a target DNA sequence, which has a wide range of applications from molecular cloning to mapping epigenetic modifications on the DNA sequence. Restriction enzymes are produced by bacteria as a defence mechanism against bacteriophage (Arber, W. (1965) Ann. Rev. Microbiol. 19, 365-378). Thus, they can be isolated from their native *E. coli* or cloned for production as recombinant proteins.

Restriction enzymes useful for targeted cleavage of nuclease Hypersensitive sites are required to cut at a defined position or within their recognition sequence (Type II restriction enzymes).

The first sequence specific restriction enzyme in accordance with the present invention may be any restriction enzyme that introduces a staggered cut (or overhang).

In one embodiment the sequence specific restriction enzyme may be one or more from the group selected from N1AIII, Fael or Hsp92II.

In one embodiment the first sequence specific restriction enzyme may be N1AIII which produces a 4 base overhang, 5'...CATG...3', at the target sequence:
5'...CATG*... 3'
3'...*GTAC... 5' where * represents the cleavage site I.

When contacting the nucleic acid sample or genomic DNA with the first sequence specific restriction enzyme, conditions are preferably selected to minimise, or more preferably exclude, reaction of the restriction enzyme with its recognition sequence located within non hypersensitive sites which, despite reduced sensitivity can react under certain circumstances.

This can be achieved by reducing the reactivity of the restriction enzyme by restricting the contact time with the intact chromatin, conducting the reaction at a sub optimal, reduced temperature for the restriction enzyme or reducing the concentration of the restriction enzyme.

The amount of nuclease used and the time of the cleavage or fragmenting steps by the first and/or second sequence specific restriction enzymes is such that the when the digestion is completed no clear banding pattern can be observed if the digested sample is analysed by electrophoresis. A skilled person using their skill and knowledge can determine the preferred concentration and exposure time for the first and/or second sequence specific restriction enzymes.

The skilled person can determine preferred concentration and exposure times by running a time course and checking the degree of degradation using pulsed field gel electophoresis. As one skilled in the art will be aware the preferred concentrations and exposure times may be cell type dependent and so can be predetermined prior to carrying out the high throughput processing of samples in accordance with the present invention.

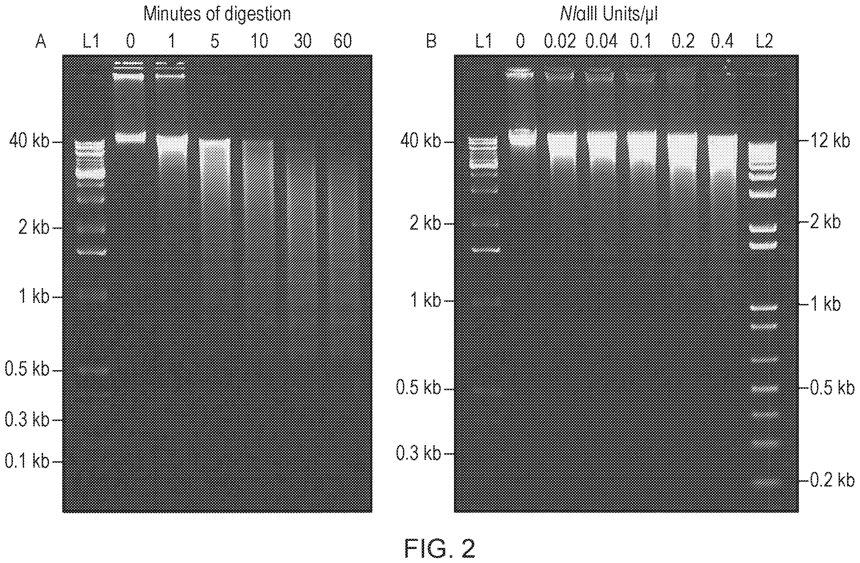

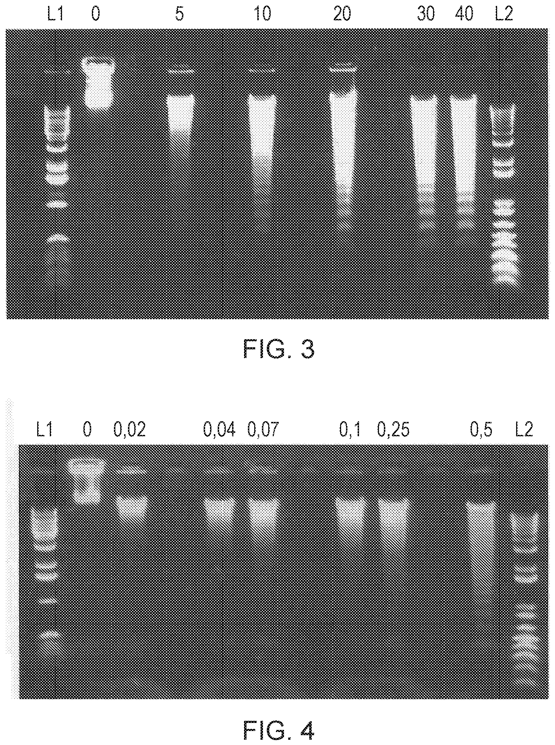

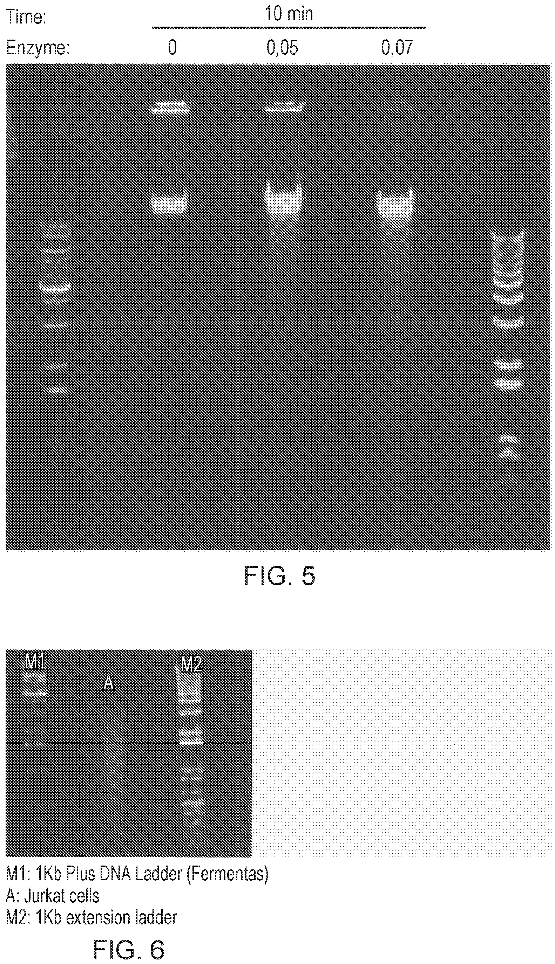

The results confirm that fine control of the primary digestion can be achieved through control of reaction time (Figure 2A, Figure 3), enzyme concentration (Figure 2B, Figure 4) and optimised to produce fragments largely devoid of non-specific digestion (Figure 5). Overdigested samples clearly display fragmentation DNA degradation patterns characterised by bands of oligonucleosome repeats (Figure 6)

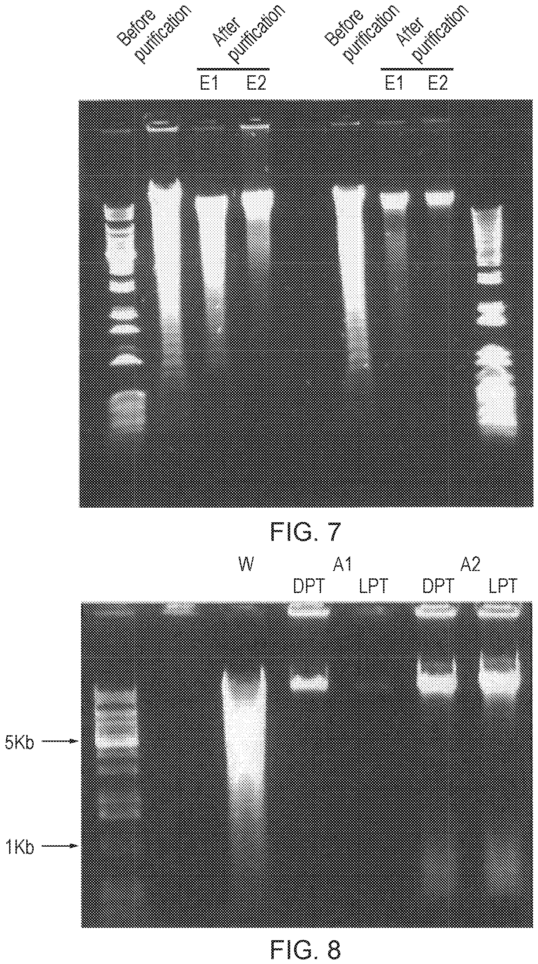

The method of the present invention may further include methods for removal of protein components from the primary nucleic acid sample digests (e.g. the cleaved or fragments nucleic acid or genomic DNA post-exposure to the first sequence specific restriction enzyme). DNA can be substantially purified from protein components following primary digestion by methods known in that art. We present data to confirm successful purification of primary digestion fragments by methods including, but not restricted to, phenol chloroform extraction and extraction with commercially available kits including DNA spin columns (Figure 7) and pippette tips (Figure 8) containing DNA binding matrices following treatment with RNAse and protease.

The method of the present invention may further include methods for removal of RNA components from the primary nucleic acid sample digests by treatment with an RNAsew according to mnethods known in the art.

Native restriction enzymes can exhibit off target, or star activity, outside their optimal reaction conditions but can be engineered to improve specificity (i.e. reduce off target cleavage). Enzymes exhibiting low star activity and improved performance over wider reaction conditions are desirable for the present invention where control of reaction conditions is required to avoid over digestion of the sample nucleic acid sequences during the primary cleavage of target sequences within the Nuclease Hypersensitive sites (for an example of an over digested sample nucleic acid sequence see Figure 6). Low star activity restriction enzymes have been developed and are available commercially e.g. NEB's range of Hi Fidelity (HF™) product line.

When the adapter oligonucleotide sequence is biotinylated, the adapter oligonucleotide may be designed and generated by annealing a biotinylated forward strand with a shorter complementary non-biotinylated reverse strand to generate a mono-biotinylated adapter with the required complementary sticky end.

The second restriction enzymes used in the present invention are required to cut at sites away from their recognition site. Type 1 restriction enzymes cut at random sites far away from their recognition sequence and thus do not produce fragments with a discrete size. These type 1 restriction enzymes are therefore not second sequence specific restriction enzymes in accordance with the present invention.

Type IIG restriction enzymes cleave at a site distal to their recognition sequences and have a target recognition domain that is separate from the catalytic site responsible for DNA cleavage. Thus, Type IIG restriction enzymes can be rationally engineered with new target sequence specificities.

Type IIG restriction enzymes can be divided into those that recognise a continuous sequence, cutting on just one side of that sequence and those that recognise discontinuous sequences and excise that entire sequence by cleaving on both sides of it.

In a preferred embodiment the second sequence specific restriction enzyme cuts at a position at a defined number of bases distal to said recognition site, wherein the defined number of bases may be between 16 and 50bp.

In a preferred embodiment the second sequence specific restriction enzyme is a Type IIG restriction enzyme.

In a preferred embodiment the Type IIG restriction enzyme used in accordance with the present invention cleaves at only one point distal to its recognition site is used.

Examples of Type IIG restriction enzymes that may be used in accordance with the present invention include, but are not limited to MmeI TCCRAC(20/18), AcuI CTGAAG(16/14), BbsI GAAGAC(2/6), BbvI GCAGC(8/12), BccI CCATC(4/5), BceAI ACGGC(12/14), BCiVI GTATCC(6/5), BcoDi GTCTC(1/5), BfuAI ACCTGC(4/8), BpuEi CTTGAG(16/14), BseRI GAGGAG(10/8), BsgI GTGCAG(16/14), BsmAI GTCTC(1/5), BSMBi CGTCTC(1/5), BSMFI GGGAC(10/14), BspCNI CTCAG(9/7), BSPQI GCTCTTC(1/4), EcoP15) CAGCAG(25/27), FokI GGATG(9/13), HgaI GACGC(5/10), HphI GGTGA(8/7), HpyAV CCTTC(6/5), MboII GAAGA(8/7), NmeAIII GCCGAG(21/19), SapI GCTCTTC(1/4).

Those skilled in the art will appreciate that combining a DNA cleavage domain with a protein capable of targeting a specific DNA sequence (such as a zinc finger DNA-binding domain) can be used to generate a synthetic enzyme (such as a Zinc Finger Nuclease).

Thus in one embodiment a synthetic enzyme may be employed provided that the enzyme introduces a cut with an overhang or sticky end capable of distinguishing between a targeted cut and a random break within the nuclease hypersensitive sites.

It will be clear to those skilled in the art that the size of nucleic acid fragments produced by the method reported herein is dependent on the second sequence specific restriction enzyme that cuts at a specific distance distal from its recognition site introduced within the first adapter oligonucleotide. The distance that the sequence specific restriction enzyme cuts from its recognition site is preferably between about 16-50 bp, more preferably between about 16-33 bp cutter, more prefererably between about 18-33, and most preferably between about 20-33.

In a preferred embodiment the sequence specific restriction enzyme that cuts at a specific distance from its recognition site is MmeI.

Mme1 cuts specifically 20bp from its recognition site leaving a 2 base overhang. The target sequence of *MmeI* is:
5'...TCCRAC(N)₂₀*...3' where R is A or G
3'...AGGYTG(N)₁₈*...5' where * represents the cleavage site and Y is T or C
Thus in a preferred embodiment embodiment where the primary Nuclease Hypersensitive sites are targeted with N1AIII and the secondary restriction enzyme is Mme1, the primary adapter sequence is a modified version of the Illumina NIaIII gene expression oligonucleotide sequence *Bio
tin- 5'...ACAGGTTCAGAGTTCTACAGTCCGACATG...3'
*3'............CAAGTCTCAAGATGTCAGGCT_{-P}.........5'

Ligation of the preferred adapter nucleotide to the sticky ended primary N1AIII cleavage products in the nucleic acid sample completes the target sequence for Mme1 targeting.

In one embodiment the secondary digestion is carried out in aqueous medium following ligation of the first adaptor and followed by purification of the digested fragments on an affinity matrix via the affinity tag on the primary adaptor.

Surprisingly we found that bound nucleic acid sequences can be digested on the affinity matrix. This facilitates sample handling, particularly in automated systems. Therefore in one embodiment the nucleic acid sequences with ligated first adaptors may be first bound via the affinity tag on the primary adaptor and may be digested on the affinity matrix.

It will be clear to one skilled in the art that the cleavage products for the Mme1 enzyme will have non-specific 2 base overhang sticky ends i.e. sequences containing each possible combination of 2 base overhang.

Thus in a preferred embodiment the secondary adapter contains degenerate 2 base overhangs to allow specific ligation to the secondary restriction digest products. In some embodiments, the second adapter may contain a primer sequence complementary to the first primer sequence introduced via the primary adapter.

In one embodiment the second adaptors may be ligated to the digested fragments on the affinity matrix followed by purification and PCR amplification from the bead.

In one embodiment the affinity matrix is a bead more preferably a magnetic bead and most preferably a streptavidin coated magnetic bead.

In one embodiment the affinity matrix is within the tip of a pipette (for example Thermo Scientific's Disposable Automated Research Tips), and more preferably a streptavidin-coated matrix within the tip of a pipette.

Thus, in a preferred embodiment all steps following ligation of the primary adaptor oligonucleotide through to PCR amplification of defined length nucleic acid sequences are performed on an affinity matrix, preferably a biotinylated matrix, more preferably a biotinylated bead and most preferably a biotinylated magnetic bead.

In a second preferred embodiment all steps following ligation of the primary adaptor oligonucleotide through to PCR amplification of defined length nucleic acid sequences are performed on an affinity matrix, preferably a biotinylated matrix, and most preferably a biotinylated matrix within a pipette tip for example Thermo Scientific's MSIA Streptavidin Disposable Automated Research Tips (Kiernan et al. Thermo Fisher Scientific Application note MSIA1004; 2013)

A method of isolating the nucleic acid sequences from Nuclease Hypersensitive sites according to the present invention followed by
i) sequencing of the resulting nucleic acid sequences; and
ii) analysing the sequence data to identify relative accessibility of the nuclease hypersensitive sites is taught herein.

It will be clear to those skilled in the art that libraries of nucleic acid sequences can be sequenced using first generation Sanger sequencing however improvements in sequencing technology are continuing to offer enhanced throughput and capability. Examples include, but are not limited to, Next Generation sequencing by synthesis including reversible dye termination (Illumina) pyrosequencing (e.g. Life Ssciences), sequencing by ligation (SOLiD) as well as next Next Generation sequencing using pH Chip (Ion Torrent) and pore based approaches (Oxford Nanopore) which offer single molecule sequencing approaches.

Thus, in one embodiment nucleic acid sequences preferably derived from a multitude of genes and more preferably from a genome wide Nuclease Hypersensitive sites are sequenced using the Illumina platform, preferably the GAIIx, more preferably the HiSeq 1000, more preferably the HiSeq 1500, more preferably the HiSeq 2000 more preferably the HiSeq 2500.

In one embodiment sequencing data is analysed using a pipeline of bioinformatics tools. An example of the pipeline used in the present invention is given in Figure 9. This proprietary method allows sequencing of HyperSensitive Sites libraries and is referred to herein as Hyper-Seq™. It will be clear to those skilled in the art that alternative and additional bioinformatics tools can be applied to the analysis of the sequencing data.

A method of isolating the nucleic acid sequences from Nuclease Hypersensitive sites according to the present invention followed by
i) applying the resulting nucleic acid sequences to a whole genome tiled array, and
iii) analysing the microarray data to identify relative accessibility of the nuclease hypersensitive sites is taught herein.

The term "aqueous medium" or "aqueous solution" is defined herein means one which is non-gelling. Suitably the aqueous medium or aqueous solution comprises water (H₂0) as the solvent. The aqueous solution may incorporate dissolved electrolytes (ionic substances) or non-electrolytes (non dissociative solutes) but importantly for the present invention no or substantially no polymeric material (e.g. low melting point agarose) or other gelling agents. The aqueous solution will remain liquid until it reaches its freezing point and will not exhibit a gel transition temperature

In one embodiment, the term "aqueous medium" as used herein means a medium in which the movement of protein, for example a restriction enzyme, a protease or an RNAse or DNA fragment is not inhibited, for example by addition of a polymeric gelling agent.

By "movement not being inhibited" as used herein means that the movement compared with that seen in water.

In one embodiment the term "aqueous solution" as used herein means a medium which comprises less than 5g/L polymeric material (e.g. agarose), and preferably no or substantially no polymeric material.

The term "substantially no" means less than 2g/L polymeric material (e.g. agarose) or other gelling material.

In one embodiment the term "aqueous medium" as used herein means a medium which comprises less than 5g/L gelling agent.

Aqueous buffers are selected to optimize reactivity and minimize off target activity of the restriction enzyme. Commercially available buffers have been developed for optimized performance of specific enzymes. Examples from New England Biolabs Inc. include NEBuffer 1 (10mM Bis-Tris-Propane-HCl 10mM MgCl₂, 1mM DTT, pH 70@25°C) NEBuffer 1.1 (10mM Bis-Tris-Propane-HCl, 10mM MgCl₂, 100µg/ml BSA, pH 7.0@25°C); NEBuffer 2.1 (50mM NaCl, 10mM Tris-HCl,10mM MgCl₂,100µg/ml BSA, pH 7.9@25°C); NEBuffer 3.1 (100mM NaCl, 50mM Tris-HCl, 10mM MgCl₂,100µg/ml BSA, pH 7.9@25°C): NEBuffer 4 (50mM Potassium Acetate, 20mM Tris-acetate, 10mM Magnesium Acetate, 1mM DTT, pH 7.9@25°C); and CutSmart Buffer (50mM Potassium Acetate, 20mM Tris-acetate, 10mM Magnesium Acetate, 100µg/ml BSA, pH 7.9@25°C)

It will be clear to one skilled in the art that the activity of specific enzymes will vary depending on the buffer and temperature used. Selection of the correct buffer is essential; for example, Restriction enzyme NiAIII has an activity of 10% in NEBuffer 1.1, 1.2 and 1.3 compared to 100% activity in 1x Cutsmart buffer at 37°C, Restriction enzyme MMel NiAIII has an activity of 50% in NEBuffer 1.1, 100 % in NEBuffer 1.2 and 50% activity in NEBuffer 1.3 compared to 100% activity in 1x Cutsmart buffer at 37°C the Cutsmart buffer system was designed as a generic bufefer system for over 200 enzymes available from New England Biolabs Inc,

The methods according to the present invention may be utilized to creat a library, e.g. comprising a collection of polynucleotides corresponding to HyperSensitive (HS) site regions (e.g. accessible regions of cellular chromatin). The libraries can be prepared from chromatin samples from, for example, cells at different stages of development, different tissues, from diseased and counterpart healthy cells, and/or infected cells and counterpart uninfected cells.

The polynucleotide fragments prepared by the method of the present invention can be sequenced and the resulting sequences used to populate a database. Such databases can include other information relevant to the isolated polynucleotide sequences, such as type of cell the sequences were isolated from for example. The database can include sequences for polynucleotide sequences from a single sample of cellular chromatin or sequences from multiple samples. The database can include sequences for polynucleotide fragments isolated from, for example, cells at different stages of development, different tissues, from diseased and counterpart healthy cells, and/or infected cells and counterpart uninfected cells.

In one embodiment the present invention may also provide a computer system that generally includes a database and a user interface. The database in such systems comprises sequence records that include an identifier that identifies one or more projects to which each of the sequence records belong. The system may include a processor operatively disposed to (i) compare one or more polynucleotide sequences from each of a plurality of collections of polynucleotide sequences, wherein each collection comprises a plurality of polynucleotide sequences corresponding to the HSS from a nucleotide sequence comprising chromatin (e.g. genomic DNA or cellular chromatin), different collections comprising polynucleotide sequences that correspond to HSS for different samples of a nucleotide sequence comprising chromatin (e.g. genomic DNA or cellular chromatin); (ii) identifying one or more polynucleotides unique or common to at least one of the plurality of collections; and (iii) display the identified polynucleotide sequence(s).

Epigenetic control of genome accessibility through chromatin structuring is a key aspect of cell function and consequently dysfunction. HS mapping is therefore a valuable tool for profiling cells including, but not limited to, characterization of tissue samples, peripheral blood samples or cells grown in tissue culture including normal differentiated primary cells, immortalized primary cells and malignancy derived cell lines, and stem cells.

Examples of potential applications include, but are not limited to, evaluation of consistency of a cell line over a number of passages (e.g. for diagnostic use); determination of differentiation status; discovery of biomarkers for specific disease indications; identification of targets for drugs; to assess the affect of drugs or other molecules or procedures on cells for diagnostic, prognostic or treatment selection purposes; to identify drugs that interact with a target identified by the method; and identifying open regions (or HSS) that are associated with a disease (e.g. by comparing diseased state with healthy state).

### DISEASE PROGRESSION

Disease progression may be associated with changes in chromatin structure in affected cells. Thus, in addition to diagnosing diseases, the present invention may also be used to monitor the progress of a disease in a subject. For example, the progression of a particular type of cancer afflicting a subject may be determined by determining the chromatin structure (e.g. HSSs) in the subject's diseased cells and comparing them with chromatin structures (e.g. HSSs) indicative of the progression of a particular type of cancer. As indicated previously, for convenience the comparison may best be carried out using a library of HSSs - such as a collection of HSSs in a computer database of fragments generated using the methods of the present invention.

### CELLULAR DEVELOPMENT

Chromatin structure may also be an indicator of cellular development. In this regard, cells at different stages of development have unique chromatin structures and hence different HSSs. Thus, the present invention may be used to monitor cell development in a cell population.

Multiple samples may be taken to enable cell development, disease progression or efficacy to be determined. In such situations the determination is relative, based on differences in HSSs between samples. However, it will be appreciated that the same result can be achieved by comparing the fragment pattern from a test sample with a library of HSSs - such as a collection of HSSs in a database of fragment fingerprints indicative of cellular development.

### CHROMATIN MODIFICATION

The methods of the present invention facilitate the generation of a substantial amount of information on chromatin structure and more particularly the location, sequence and role of HSSs within chromatin. Once the sequence and role of particular HSSs has been determined using the present invention, they may be modified to alter the expression of genetic information from chromatin.

The nucleic acid sequences in chromatin may be modified using standard techniques, such as site directed mutagenesis, to either include or remove one or more HSSs. These modifications to the nucleic acid sequence will in turn affect the HSSs and chromatin structure and the expression of genetic information therein.

As an alternative to modulating (e.g. modifying) chromatin structure by altering the nucleic acid sequence, chromatin may be modified using agents that act in a more general fashion to cut and reshape chromatin (and hence the HSSs) without necessarily altering individual nucleotides. In this regard, the present invention also enables the identification and characterisation of such chromatin modulating (e.g. modifying) agents. More particularly, the ability of the methods of the invention to provide information on chromatin structure facilitates the screening of potential new chromatin modulating (e.g. modifying) agents and enables known agents to be better characterised.

Thus, the present invention may also be used to identify one or more agents capable of modulating (e.g. modifying) chromatin structure. Preferably, the agents act directly on the chromatin in the sample to modify its structure by binding to the chromatin and affecting one or more HSSs. Alternatively, the agent may affect the formation or expression of HSSs in the chromatin.

As well as identifying chromatin modulating (e.g. modifying) agents, the present invention also enables the identification of binding sites for chromatin modulating (e.g. modifying) agents. In this regard, the present invention may be used for identifying chromatin modulating (e.g. modifying) agent binding sites. Preferably, the chromatin modulating (e.g. modifying) agent is selected from the group comprising oestrogen.

### CHROMATIN STRUCTURE

Chromatin structure reflected by the HSs therein affects the expression of the encoded nucleic acid and in turn the functioning of the cell. The methods of the present invention facilitate the control of cellular functions by modulating (e.g. modifying) chromatin structure and thus the expression of the genetic information. Thus, the present invention may also be used to treat a nucleic acid sample to control its expression.

The pre-determined form may be any chromatin structure that has an effect on the functioning of a cell containing the chromatin. The predetermined form may be a structure that predisposes a cell to differentiate in a particular way. In this regard, the invention may be used to prepare customised cell populations from progenitor cells. For example, once the chromatin structure that predisposes a cell to differentiate in a particular way has been determined, progenitor cells may be treated to modify their chromatin structure as necessary to predispose cells to differentiate into particular cell types. The control of differentiation by modulating (e.g. modifying) chromatin structure enables the production of any desired cell population or the production of a uniform progenitor population with the ability to differentiate into a given cell type or types. This form of the invention may have particular application in embryonic and somatic stem cell therapy as it enables monitoring of the uniformity of the differentiation state of cell populations for administration to subjects to maximise the effectiveness of the therapy. By monitoring chromatin states it may also be possible to devise protocols capable of guiding undifferentiated embryonic stem cells into specified differentiation pathways in a stepwise and controlled manner.

The predetermined form may also be a chromatin structure that is capable of expressing a nucleic acid sequence contained therein in a preferred fashion relative to unmodified chromatin. This form of the invention may be particularly useful where the expression of the gene of interest is maximised for therapeutic purposes, such as in gene therapy.

As an extension to this form of the invention, the present invention is particularly useful in the design and production of gene constructs, including those used for gene therapy applications and in transgenics. In this regard, in addition to other regulatory and control sequences in the construct, the present invention enables a skilled person to design a construct adapted for optimal presentation in the chromatin to which it is inserted. Constitutive HSSs may serve as border elements that define functional chromatin domains or may facilitate the precise folding patterns of individual chromatin fibres. Thus, constructs designed for optimal presentation in the chromatin will define one or more HSSs that will ensure correct chromatin structure and in turn enable the most efficient expression of the inserted nucleic acid.

For therapeutic applications the predetermined form may also be a chromatin structure that corresponds to a non-disease phenotype. In this regard, chromatin modulating (e.g. modifying) agents may also be used to treat diseases related to chromatin structure. For example, cancer may be treated by administering a chromatin modulating (e.g. modifying) agent that modifies the chromatin in a cancer cell to prevent it from uncontrolled division. The particular agents used to modify the chromatin for therapeutic purposes will depend on the nature of the chromatin changes required. However, once the chromatin structure corresponding to a diseased phenotype has been identified using the methods of the present invention, agents may be selected that are adapted to alter particular aspects of chromatin structure for therapeutic benefit.

### AGENTS

The agents identified using the method of the present invention may be used for diagnostic purposes (i.e. a diagnostic agent) and/or for therapeutic purposes (i.e. a therapeutic agent).

The agent may be an organic compound or other chemical. The agent may be a compound, which is obtainable from or produced by any suitable source, whether natural or artificial. The agent may be an amino acid molecule, a polypeptide, or a chemical derivative thereof, or a combination thereof. The agent may even be a polynucleotide molecule - which may be a sense or an anti-sense molecule. The agent may even be an antibody.

### THERAPEUTIC AGENTS

The present invention may be used to test therapeutic agents that effect chromatin structure in a subject. For example, the chromatin structure in a subject administered with a therapeutic agent may be determined by determining the chromatin structure in the subject's cells and comparing them with chromatin structures from a subject not being tested with the therapeutic agent. As mentioned previously, for convenience the comparison may best be carried out using a HS library such as a computer database of fragment patterns generated using the methods of the present invention.

Furthermore, the present invention may be used to monitor the efficacy of a therapeutic agent capable of treating a disease in subject.

### CHROMATIN MODULATING AGENT

The methods of the present invention may be used to identify one or more agents that modulate (e.g. modify) chromatin, compositions for use in medicine comprising at least one chromatin modulating (e.g. modifying) agent of the present invention and methods of using chromatin modulating (e.g. modifying) agents of the present invention in the preparation of a medicament for the treatment of diseases.

As used herein, the term "chromatin modulating agent" may refer to a single entity or a combination of entities.

The chromatin modulating agent may be an organic compound or other chemical. The chromatin modulating agent may be a compound, which is obtainable from or produced by any suitable source, whether natural or artificial. The chromatin modulating agent may be an amino acid molecule, a polypeptide, or a chemical derivative thereof, or a combination thereof. The chromatin modulating agent may even be a polynucleotide molecule - which may be a sense or an anti-sense molecule. The chromatin modulating agent may even be an antibody.

The chromatin modulating agent may be designed or obtained from a library of compounds, which may comprise peptides, as well as other compounds, such as small organic molecules.

By way of example, the chromatin modulating (e.g. modifying) agent may be a natural substance, a biological macromolecule, or an extract made from biological materials such as bacteria, fungi, or animal (particularly mammalian) cells or tissues, an organic or an inorganic molecule, a synthetic agent, a semi-synthetic agent, a structural or functional mimetic, a peptide, a peptidomimetics, a derivatised agent, a peptide cleaved from a whole protein, a peptide synthesised synthetically (such as, by way of example, either using a peptide synthesizer or by recombinant techniques) or combinations thereof, a recombinant agent, an antibody, a natural or a non-natural agent, a fusion protein or equivalent thereof and mutants, derivatives or combinations thereof.

The chromatin modulating (e.g. modifying) agent may be an organic compound. Typically the organic compounds may comprise two or more hydrocarbyl groups. Here, the term "hydrocarbyl group" means a group comprising at least C and H and may optionally comprise one or more other suitable substituents. Examples of such substituents may include halo-, alkoxy-, nitro-, an alkyl group, a cyclic group etc. In addition to the possibility of the substituents being a cyclic group, a combination of substituents may form a cyclic group. If the hydrocarbyl group comprises more than one C then those carbons need not necessarily be linked to each other. For example, at least two of the carbons may be linked *via* a suitable element or group. Thus, the hydrocarbyl group may contain hetero atoms. Suitable hetero atoms will be apparent to those skilled in the art and include, for instance, sulphur, nitrogen and oxygen. The chromatin modulating (e.g. modifying) agent may comprise at least one cyclic group. The cyclic group may be a polycyclic group, such as a non-fused polycyclic group. The chromatin modulating (e.g. modifying) agent may comprise at least one of said cyclic groups linked to another hydrocarbyl group.

The chromatin modulating (e.g. modifying) agent may contain halo groups. Here, "halo" means halogen compounds eg. halides and includes fluoro, chloro, bromo or iodo groups.

The chromatin modulating (e.g. modifying) agent may contain one or more of alkyl, alkoxy, alkenyl, alkylene and alkenylene groups - which may be unbranched- or branched-chain.

The chromatin modulating (e.g. modifying) agent may be in the form of a pharmaceutically acceptable salt - such as an acid addition salt or a base salt - or a solvate thereof, including a hydrate thereof. For a review on suitable salts see Berge et al, J. Pharm. Sci., 1977, 66, 1-19.

The chromatin modulating (e.g. modifying) agent of the present invention may be capable of displaying other therapeutic properties.

The chromatin modulating (e.g. modifying) agent may be used in combination with one or more other pharmaceutically active agents.

If combinations of active agents are administered, then they may be administered simultaneously, separately or sequentially.

In one embodiment the method relates to the identification of an agent for the treatment of a disease, e.g. as exemplified by the identification of oestrogen for the treatment of breast cancer.

### ADVANTAGES

The present inventions has many advantages over prior art methods.

In some embodiments the present invention provides a library where there is low noise or where the background noise has been significantly reduced.

The present invention represents a simplified process which is also quicker.

The present invention is suitable for automation e.g. by liquid handling robots, which allows high throughput of samples. It is also possible to run multiple samples in parallel, thus again speeding up the processing time.

In addition the present invention may be cheaper.

In addition or alternatively, the present invention leads to no (or minimal) loss of data due to removal of randomly fragmented nucleic acids or size fractionation

As indicated above, hypersensitive sites are an important regulatory access point for external agents to act upon the genome. Thus, it will be appreciated that the methods of the present invention have many applications in biotechnology and medicine. The methods of the present invention are broadly applicable to all eukaryotic genomes and allow for the profiling of one or more cells, one or more nuclei or one or more tissue samples based on their chromatin structure. The methods of the present invention are also broadly applicable to all eukaryotic genomes and allow for the profiling of one or more isolated cells, one or more isolated nuclei or one or more isolated tissue samples based on their chromatin structure. In particular, chromatin from certain diseased cells, nuclei, or tissues has an altered chromatin structure relative to the chromatin from otherwise healthy cells.

According to the methods of the present invention, a disease associated with an altered chromatin structure may be diagnosed in a subject. The most convenient way to diagnose the disease is to compare the fragments from a hypersensitive site library - such as a collection of hypersensitive sites comprising a database of fragments indicative of the disease. Preferably, the disease associated with altered chromatin structure is selected from the group consisting of: cancer, chronic diseases, aging and genetic diseases.

Furthermore, when particular diseases have characteristic chromatin structures, the methods of the present invention may be used to diagnose the particular form or type of a disease. For example, the particular form of cancer afflicting a subject may be determined by determining the chromatin structure in the subject's diseased cells and comparing them with chromatin structures indicative of particular forms of cancer. As indicated previously, for convenience the comparison may best be carried out using a HS library - such as a collection of HSs comprising a computer database of fragment patterns generated using the methods of the present invention. The detailed and accurate diagnosis of disease forms such as cancer facilitates the correct choice of therapeutic treatment for the disease and thus increases the chances of successfully treating the disease.

Unless defined otherwise, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this disclosure.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino to carboxy orientation, respectively.

The headings provided herein are not limitations of the various aspects or embodiments of this disclosure which can be had by reference to the specification as a whole. Accordingly, the terms defined immediately below are more fully defined by reference to the specification as a whole.

Amino acids are referred to herein using the name of the amino acid, the three letter abbreviation or the single letter abbreviation.

The term "protein", as used herein, includes proteins, polypeptides, and peptides.

As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "enzyme".

The terms "protein" and "polypeptide" are used interchangeably herein. In the present disclosure and claims, the conventional one-letter and three-letter codes for amino acid residues may be used. The 3-letter code for amino acids as defined in conformity with the IUPACIUB Joint Commission on Biochemical Nomenclature (JCBN). It is also understood that a polypeptide may be coded for by more than one nucleotide sequence due to the degeneracy of the genetic code.

Other definitions of terms may appear throughout the specification. Before the exemplary embodiments are described in more detail, it is to understand that this disclosure is not limited to particular embodiments described, as such may, of course, vary. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise.

The publications discussed herein are provided solely for their disclosure prior to the filing date of the present application. Nothing herein is to be construed as an admission that such publications constitute prior art to the claims appended hereto.

### ISOLATED

In one aspect, preferably the DNA is isolated. The term "isolated" means that the DNA is at least substantially free from at least one other component with which the DNA is naturally associated in nature and as found in nature. The DNA of the present invention may be provided in a form that is substantially free of one or more contaminants with which the substance might otherwise be associated. Thus, for example it may be substantially free of one or more potentially contaminating polypeptides and/or nucleic acid molecules. Preferably, the isolated DNA is present in the sample at a level of at least about 90%, or at least about 95% or at least about 98%, said level being determined on a dry weight/dry weight basis with respect to the total composition under consideration.

The invention will now be described, by way of example only, with reference to the following Figures and Examples.

### EXAMPLES

### Example 1

Rapid preparation of Nuclease Hypersensitive Site library using a solution method (e.g. a method carried out in an aqueous medium)

Previous approaches to genome wide Nuclease Hypersensitive site mapping have either resulted in high background signal, loss of signal or extended processing times due to specific processing steps undertaken to reduce non-specific cleavage of the larger nucleic acid sequences generated following primary digestion within the Nuclease Hypersensitive sites. This includes processing primary digest fragments in low melting agarose gel to prevent mechanical damage to the larger DNA fragments. Such methods are not well suited to rapid library preparation due to reduced reaction kinetics within the gel.

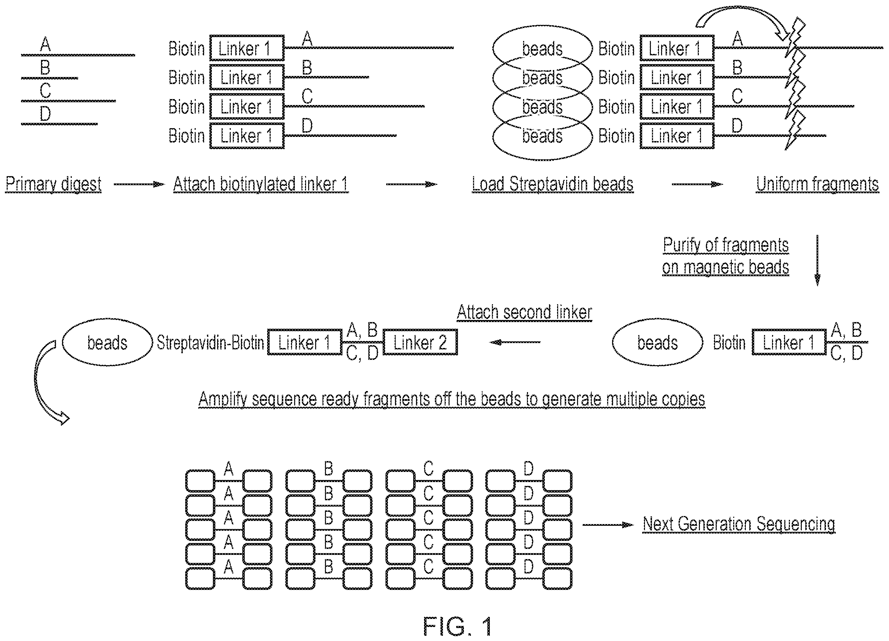

Implementation of a method for rapid Genome Wide Nuclease Hypersensitive site library preparation is described herein. The principle is summarised in Figure 1. Nuclei are first asymmetrically cleaved at defined target points within the Nuclease Hypersensitive Sites using a restriction enzyme. A preferred primary restriction enzyme is NIaIII which introduces a 4bp sticky end. A biotinylated adapter with a primer region, a complementary sticky end and containing a second target sequence for a secondary restriction enzyme is ligated to the NIaIII cleaved nuclease Hypersensitive sites. The secondary restriction enzyme cuts within the ligated DNA sequences at a defined length distal to its recognition sequence leaving a degenerate sticky end. One example of a suitable enzyme is Mmel. A second adapter containing degenerate sticky ends and a primer complementary to the one in the first adapter is ligated to the defined length fragments. Amplification of the nucleic acid sequences by PCR followed by Next Generation Sequencing and alignment to the human genome allows the positions of the Nuclease Hypersensitive to be determined.

**Jurkat cell growth:** T-*cell* leukemia Jurkat cells were grown in RPMI + 10% fetal calf serum, in a 37°C incubator at 5% CO₂. Cells were grown in 75cm² cell culture flasks (T75 flasks) and cells were provided with fresh medium every 2 days. When cells reached 80-90% confluence, they were transferred in a 50ml conical centrifuge tube and counted. Then, the tube was centrifuged for 5min at 1000g, medium was removed and cells were rinsed with 5 ml of ice-cold Phosphate Buffer Saline (PBS). Cells were pelleted by centrifugation (5min, 1000g) and 2ml of 70% Ethanol were added to the cell pellet to freeze the epigenetic status of the cells. The cell pellet was used immediately or stored at -20°C.

**Nuclei Isolation:** Nuclei from frozen cells were extracted using the Nuclei EZ prep Nuclei isolation kit (Sigma) according to manufacturer's protocol. Ethanol was removed and the cell pellet washed in PBS. Cells were collected by centrifugation for 5min at 500g. The cell pellet was resuspended in 4ml of nuclei EZ lysis buffer. After 5 min incubation, the tube was centrifuged for 5min at 500g. This lysis step was repeated twice. Nuclei were collected by centrifugation and the nuclei pellet was then resuspended in 200µl of Nuclei EZ storage buffer. The pellet was mixed by vortexing and by pipetting and the final nuclei suspension was transferred to a microcentrifuge tube. A small fraction was taken for counting (cell counter, Bürker). Nuclei were used immediately or frozen at -80°C for storage.

Isolated nuclei were digested with the restriction enzyme NlaIII in order to identify Nuclease Accessible Sites (NAS). NAS are typically 100x more sensitive (hypersensitive) to DNAsel than DNA in condensed regions. NlaIII restriction enzyme digestion enhances specificity and introduces a primary tag for ligation of the first of two sequencing adapters. Digestion conditions need to be carefully optimized to prevent "over-digestion" away from the primary site and "star" activity of the enzyme i.e. non-specific cuts. Over digestion is the principle concern, as this will introduce false signals. In the case of star activity, subsequent purification will remove non-targeting cuts.

**Nuclei digestion (optimisation of primary digestion):** Nuclei suspension obtained according to the previous method were centrifuged for 5 min at 500g at 4°C. The cell pellet was washed twice with 1ml of 1x NEBuffer 4 (50mM Potassium Acetate, 20mM Tris-acetate, 10mM Magnesium Acetate, 1mM DTT pH 7.9 @ 25°C, New England BioLabs). Nuclei were resuspended at a density of 3x10⁶ nuclei/ml in 1mL ice-cold 1x NEBuffer 4 supplemented with 100µg/ml BSA (New England BioLabs) and then incubated for 5 minutes at 37°C.
For optimisation of the primary digest conditions aliquots of 3x10⁶ nuclei (1ml) were incubated at 37°C with 1.0U/mL of NlaIII enzyme (New England BioLabs) for varying time periods from 1 to 60 minutes (1, 5, 10 30 and 60 minutes). Digestion reaction was stopped by addition of 52µl of 0.5M EDTA (Sigma-Aldrich). 20µl of RNAse A (10mg/ml; Roche Diagnostics) and 4.8µl RNAse T1 (100U/µl, Roche Diagnostics) were added into the micro centrifuge tube and followed by incubation at 37°C for 30min. Then, 20µl of proteinase K (20mg/ml; New England Biolabs) were added and sample was incubated for 2 hours at 50°C.
Analysis by polyacrylamide gel electrophoresis showed distinct banding representing over-digestion via non-specific cuts around nucleosomes (Figure 2A). This pattern (mono and oligomeric nucleosomal DNA) was not evident at 1 min incubation however such a short time frame is not ideal from an experimental reproducibility perspective.
In a second series of experiments, aliquots of 3x10⁶ nuclei (1ml) were incubated at 37°C for 5 minutes with varying concentrations of NIaIII enzyme (0.02, 0.04, 0.1. 0.2, and 0.40U/mL). Analysis by polyacrylamide gel electrophoresis showed a broadening of the high molecular weight band at all levels with appearance of over-digestion banding at concentrations above 0.1U/ I distinct banding representing over-digestion (Figure 2B).
Higher concentration of Jurkat nuclei (3x10⁶/mL) digested with 0.1U/µL NiAIII in NEB4 resulted in over digestion at 5 minutes with the appearance of a clear banding pattern (Figure 3). Further reduction in enzyme concentration (0.02-0.04 U/µL) allowed 30-minute digestions with no banding (Figure 4).
In a third series of experiments 1mL aliquots of Jurkat nuclei were incubated at 37°C for 10 minutes with 0.05U/ mL and 0.07U/mL NiAIII in NEB4 buffer. No banding due to over-digestion was observed with a high molecular weight band clearly visible indicating optimal digestion conditions (Figure 5). 3x10⁶/mL Jurkat cells digested for 10 minutes with 0.07U/mL were selected for Nuclease Hypersensitive Site library preparation. An over digested sample was produced by treating the same number of cells for 10 minutes with 0.5U/mL. Gel electrophoresis indicated that the majority of the sample was present as oligomeric nucleosomes with no high e really molecular weight band present (Figure 6)

Primary Digest Purification: Digested DNA was purified using the Wizard SV gel and PCR clean up System kit (Promega) modified from the manufacturer's instructions. The Wizzard SV system effectively removed low molecular weight contaminants and produced highly purified DNA for subsequent steps (Figure 7) Purified digested DNA was resuspended in 50µl of buffer and quantified by adsorption at 260nm with an assessment of purity made by the 260nm/280nm ratio with a nanodrop (Thermo Scientific).

The Akkoni tru tip system (Akkoni) also produced highly purified DNA without low molecular weight contamination but with a lower yield (Figure 8)

**Adapter one ligation:** The primary adapter containing the NlaIII complementary sticky end and an Mmel target site at the 3' end was generated by annealing 10µl 5'-Bio-*ACAGGTTCAGAGTTCTACAGTCCGACATG3' with 10µl 5'P-*GTCGGACTGTAGAACTCTGAAC 3' (12.5pmol/µl) were incubated for 5min at 95°C, and slowly cooled down at 25°C. Linker could then be stored at 4°C. 3µg of digested DNA was mixed with 6µl of linker 1 (25pmol/µl), 2µl of T4 DNA ligase (5U/µl; Roche), 5µl of 10x Ligation Buffer (Roche) to a final volume of 50µl and incubated overnight at 20°C. Un-ligated linkers were removed from Ligated1-DNA by electrophoresis and purified using a Wizard SV gel and PCR clean up kit (Promega) according to the manufacturer's instructions.
*Oligonucleotide sequences © 2006 Illumina, Inc. All rights reserved. Illumina

**Off bead digestion:** 75µL of ligated 1-DNA was added to 10µL NEB buffer 4 (10x), 10µL of 500µM S-Adenosyl methionine and 5µL of MMel stock solution (2U/ µl; New England Biolabs) followed by incubation for 90 minutes at 37°C. The ligated product was dephosphorylated with 3µl Fast Alkaline phosphatase (FastAP) (3U/µl; ThermoScientific)

**Magnetic Bead preparation:** 50µl of ligated 1- DNA was added to 50µl of 2x Bind&Wash buffer (10mM Tris-CI, pH7.5; 1mM EDTA; 2M NaCl, Invitrogen). This mix was added to 100µl of magnetic beads (Dynabeads M-280 Streptavidin, Invitrogen) previously washed as described by the manufacturer. Ligated 1 DNA-beads complex was incubated for 30min at 20-25°C with shaking.

**On bead Secondary Digestion:** 50µl of ligated 1- DNA was added to 50µl of 2x Bind&Wash buffer (10mM Tris-CI, pH7.5; 1mMEDTA; 2M NaCl, Invitrogen). This mix was added to 100µl of magnetic beads (Dynabeads M-280 Streptavidin, Invitrogen) previously washed according to the manufacturer. Ligated 1 DNA-beads complex was incubated for 30min at 20-25°C with shaking. The tube was placed on a magnetic rack and the supernatant was removed. The beads were then washed 5 times with 200µl of 2x Bind&Wash buffer followed by 1 wash with 200µl of 1x NEBuffer4. 10µl of 10x NEBuffer 4, 10µl of S-adenosyl methionine (SAM) (500µM; New England Biolabs), 5µl of Mmel enzyme stock solution (2U/µl); New England Biolabs) were added to the beads immediately after the last washing step. Digestion was conducted at 37°C. After 90min, the digested sample was dephosphorylated by addition of 3µl Fast Alkaline phosphatase (FastAP) (3U/µl; ThermoScientific) with incubation for a further 90 minutes. Notably on bead secondary digestion was used as an alternative to off bead digestion.

Following either Off-bead digestion or On-bead secondary digestion the samples were analysed as follows.

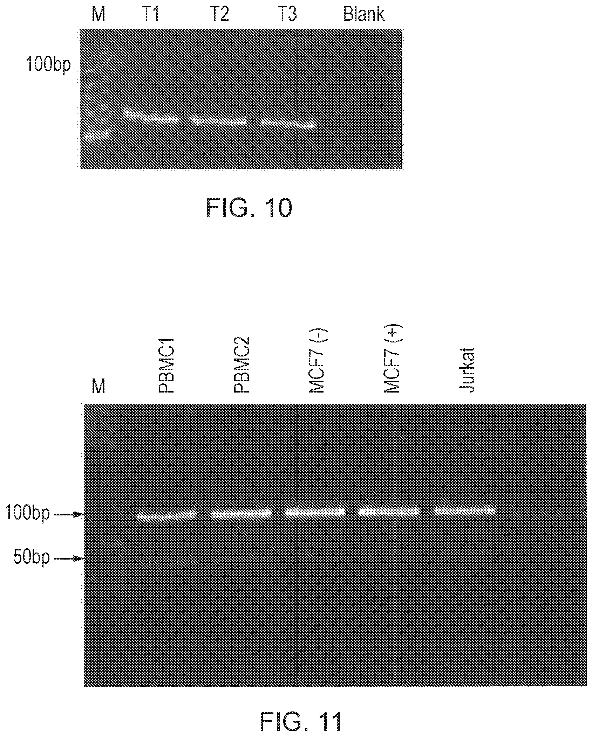

**Adapter 2 ligation:** Adapter 2 was generated analogously to adaptor one by annealing 10ml *5'CAAGCAGAAGACGGCATACGANN with 10ml *5'P-TCGTATGCCGTCTTCTGCTTG where N can be C, T, A or G and represents the degenerate sticky end (12.5mg/mL). Digested and dephosphorylated ligated 1-DNA-bead was ligated to the second adapter. The DNA-beads complex was washed once with 200µl of 1x ligation buffer (Roche) then 90µl of a ligation mix (2µl of T4 DNA ligase (5U/µl; Roche), 10µl of 10x ligation buffer (Roche), 6µl of linker 2 (25pmol/µl), 72µl of water) was added. Ligation was conducted at 20-25°C for 4 hours with gentle shaking. The ligation time could be reduced to one hour with no loss of signal following PCR amplification (Figure 10).

**PCR amplification:** Prior to amplification, the double adapter ligated DNA sequences were rendered single stranded by alkali treatment. The microcentrifuge tube containing the bead-complexed double ligated DNA sequence from the previous step was placed on a magnetic rack and the supernatant was removed and the ligated DNA-beads pellet was washed once with 1x of Bind&Wash buffer. 500µl of 0.15M NaOH was added directly on the beads for 5 min at 20-25°C with shaking. Ligated DNA-beads pellet was then washed 5 times with 200µl of 1x of Bind&Wash buffer and resuspended in 25µl of 10mM Tris-CI pH=8. Biotinylated single strand DNA was retained whilst the non-biotinylated was removed. 10µl of the bead-complexed ligated DNA was added to 40µl PCR reaction mix (1x Phusion HF Reaction buffer, 0.25µM PCR primer 1 (*5' CAAGCAGAAGACGGCATACGA), 0.25µM PCR primer 2(*5' AATGATACGGCGACCACCGACAGGTTCAGAGTTCTACAGTCCGA), 0.25mM dNTP and 1U Phusion DNA HF polymerase (New England Biolabs). The sample was denatured for 30 seconds at 98°C followed by 30 amplification cycles (10sec, 98°C; 30sec, 60°C; 15sec, 72°C) followed by extension for 7 minutes at 72°C. PCR amplicons were analysed by agarose gel electrophoresis. Two bands corresponding to the desired 86bp amplicon and PCR primers (20-30bp) were seen (Figure 11)

**Purification of Sequence Ready libraries:** 50µl of PCR product and 2µl of Ultra Low range DNA ladder (Fermentas) were loaded on a 4% agarose gel (Agarose gel Ultra Pure TM, Invitrogen; 10x TBE, Sigma; 10% Ethidium bromide, 6x orange DNA loading dye, Fermentas) After migration of the gel at 120V, the gel was placed over a UV light and the band at 86bp corresponding to the specific PCR product was excised precisely. Notably although this purification step is in a gel, this is purely a purification step and not a chemistry step. Thus the gel kinetics slowing the chemistry steps is not an issue in this purification step. In any event it is possible to circumvent the need to use a gel in this step by using exoSAP (a combination of exonuclease and shrimp alkaline phosphatase) to degrade the residual primers and bases in solution. The product can then be sequenced directly.

The excised DNA band was weighed and incubated for 10 minutes in 3x volume of QG buffer/ wt Gel (Quiquick Gel Extraction kit, Qiagen) in a 2mL microtube at 500C until dissolved. The tube was vortexed every 2 minutes to aid dissolution. 1 gel volume of isopropanol was added and the solution placed in a QIAquick spin column with a 2mL collection tube followed by centrifugation for 1 minute. The flow through was discarded and the column washed once with 0.5mL buffer QG discarding the flow through. The column was washed one with 0.75mL of buffer PE discarding the flow through and allowed to rest for 5 minutes. The column was spun for a final timefor 1 minute (17,900 g) to remove residual wash buffer then transferred to a clean 1.5mL microcentrifuge tube. Sequence ready DNA was eluted with 50mL of buffer EB (10mM Tris.HCl, pH 8.5).

Nuclease Hypersensitive site libraries were prepared according to the previous steps for two biological repeat sets of Jurkat cells. The first set (3x10⁶ nuclei) was digested with 0.05U/ L NIaIII for 10 minutes followed by off bead secondary digestion, on bead ligation of secondary adapter and PCR amplification. The second set (3x10⁶ nuclei) were treated with 0.07U/ L NIaIII followed by on bead digestion, ligation and PCR amplification. A third set (3x10⁶ nuclei) was over digested with 0.5U/mL NIaIII.

### Example 2

Rapid preparation of differential Nuclease Hypersensitive site libraries from Peripheral Blood Mononuclear Cells (PBMCs)

**Peripheral Blood Mononuclear Cell isolation (a):** 100mL of whole blood was collected from a consented, anonymysed healthy volunteer by a contract research organisation (Clinical Trials Laboratory Services, UK).
All samples are screened and confirmed negative for hepatitis B&C and HIV 1&2. Donor urine is tested for 10 of the most common drugs of abuse and samples testing positive are rejected. Donors are fasted for a minimum of 4 hrs and plasma is non-lipademic. Volunteers self certify that they have taken no medication 1 week before fasting.

Samples were collected in 8mL BD Vacutainer® CPT™ Cell Preparation Tubes with Sodium HeparinN, an evacuated Tube intended for the collection of whole blood and the separation of mononuclear cells. The cell separation medium is comprised of a polyester gel and a density gradient liquid. This configuration permits cell separation during a single centrifugation step. Each tube was inverted 8-10 times whilst successive tubes were collected to mix anticoagulant with the blood. After collection the tubes were stored upright at room temperature and centrifuged (swing out rotor) for a minimum of 15 minutes at 1500-18000 relative centrifugal force according to the manufacturer's instructions. After removal of approximately half of the plasma layer, the mononuclear cells were collected in a Pasteur pipette and transferred into a centrifuge tube. PBS was added to a volume of 15mL and the capped tube inverted 5 times. The tubes were centrifuged for 15 minutes at 300 RCF and the majority of supernatant aspirated taking care not to disturb the cell pellet. The cell pellet was resuspended by flicking and a further 10mL PBS added followed by capping, mixing by inversion 5 times and centrifugation for 10 minutes at 300RCF.

The separated sample of PBMCs were stored in as 18 x 1 mL aliquots in a freezing mixture (containing RPMI, Human Serum Albumin & DMSO) and shipped frozen on dry ice. PBMCs were store at -80°C until required.

Nuclease Hypersensitive site libraries for healthy PBMCs were prepared according to the steps in example 1. 3x10⁶ nuclei was digested with 0.07U/mL NlaIII for 10 minutes followed by on bead secondary digestion, on bead ligation of secondary adapter and PCR amplification.

**Serial Peripheral Blood Mononuclear Cell collection and isolation (b): 8mL whole blood** samples were collected in BD Vacutainer® CPT™ Cell Preparation Tubes with Sodium HeparinN according to the protocol above. Following the final wash step the cell pellet was resuspended in cold 70% ethanol (2.5mL 70% ethanol per initial 1mL blood) and re-pellet by centrifugation at 500g in a refrigerated centrifuge. The isolated pellet was resuspended in fresh cold 50% ethanol for storage and transportation. This method was employed to arrest epigenetic modification pathways and preserve chromatin structure during transport of the samples. Fixed cells can be stored at -20°C for up to 7 days prior to nuclei isolation and can be shipped on wet ice.

Serial samples were collected at time point 0 from 2 healthy volunteers and then at two further monthly intervals from one of the donors following a regime of diet and exercise.

Nuclease Hypersensitive site libraries were prepared according to the steps of example 1 for three serial biological sets of donor PBMCs. The first set (3x10⁶ nuclei) was digested with 0.07U/mL NIaIII for 10 minutes followed by off bead secondary digestion, on bead ligation of secondary adapter and PCR amplification.

The second and third sets were digested with 0.07U/mL NIaIII for 10 minutes followed by on bead secondary digestion, ligation of secondary adapter and PCR amplification.

### Example 3

Rapid preparation of differential Nuclease Hypersensitive site libraries from Oestrogen stimulated and non-stimulated MCF7 cells.

**MCF7 cell growth and oestrogen treatment:** Human breast cancer MCF7 cells were routinely grown in DNEM + 10% fetal calf serum, in a 37°C incubator at 5% CO2. Cells were grown in T75 flask and cells were provided with fresh medium every 2 days. To evaluate the effect of oestrogen, MCF-7 cells were grown in DNEM containing 5% charcoal- stripped FCS for 5 days before incubation with and without 10⁻⁷ M of β-oestradiol (Sigma-Aldrich) for 4hrs. Then medium was removed from both treated and non-treated cells and the cells were rinse with 10ml of ice-cold PBS. Cells were scraped from the flask in 5ml of ice-cold PBS and combined in a 15ml conical centrifuge tube. Cells were pelleted by centrifugation (5min, 1000g) and 2ml of 70% Ethanol were added to the cell pellet to freeze the epigenetic status of the cells. The cell pellet was used immediately or stored at -20°C.

Nuclease Hypersensitive site libraries were prepared for non-oestrogen stimulated and oestrogen stimulated MCF 7 cells according to the steps in Example 1. 3x10⁶ nuclei were digested with 0.07U/ L NIaIII for 10 minutes followed by on bead secondary digestion, ligation of secondary adapter and PCR amplification.

**Sequencing:** Nuclease Hypersensitive site libraries prepared in the previous examples were sequenced on the Illumina GAIIx and the HiSeq 2000 platforms using 36 cycle single-read protocols.

**Example 4** Identification of differential Nuclease Hypersensitive sites in Jurkat cell line and Peripheral Blood Mononuclear Cells (PBMCs) by rapid Genome wide screening

**Sequence data set A**: Three samples comprising Nuclease Hypersensitive site libraries from 1) Jurkat cells (3x10⁶ nuclei) digested with 0.07U/mL NIaIII for 10 minutes followed by on bead secondary digestion, on bead ligation of secondary adapter and PCR amplification, 2) PBMCs (3x10⁶ nuclei) digested with 0.07U/mL NIaIII for 10 minutes followed by on bead secondary digestion, on bead ligation of secondary adapter and PCR amplification and 3) The first timed serial sample of PBMCs (3x10⁶ nuclei) digested with 0.07U/mL NlaIII for 10 minutes followed by on bead secondary digestion, on bead ligation of secondary adapter and PCR amplification were sequenced as technical duplicates in separate lanes on an Illumina GAIIx platform (36 cycle, single read protocol).

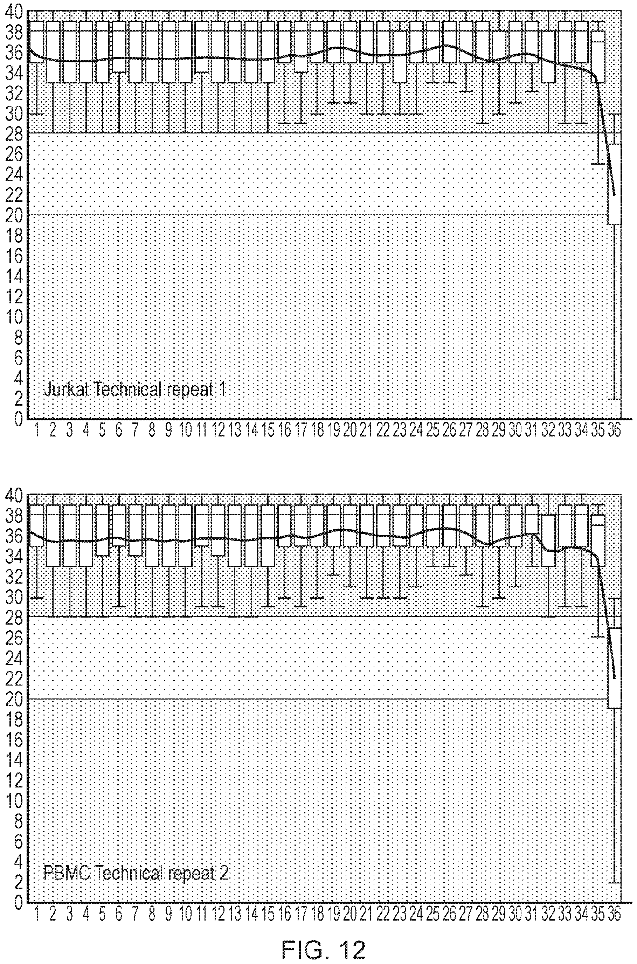

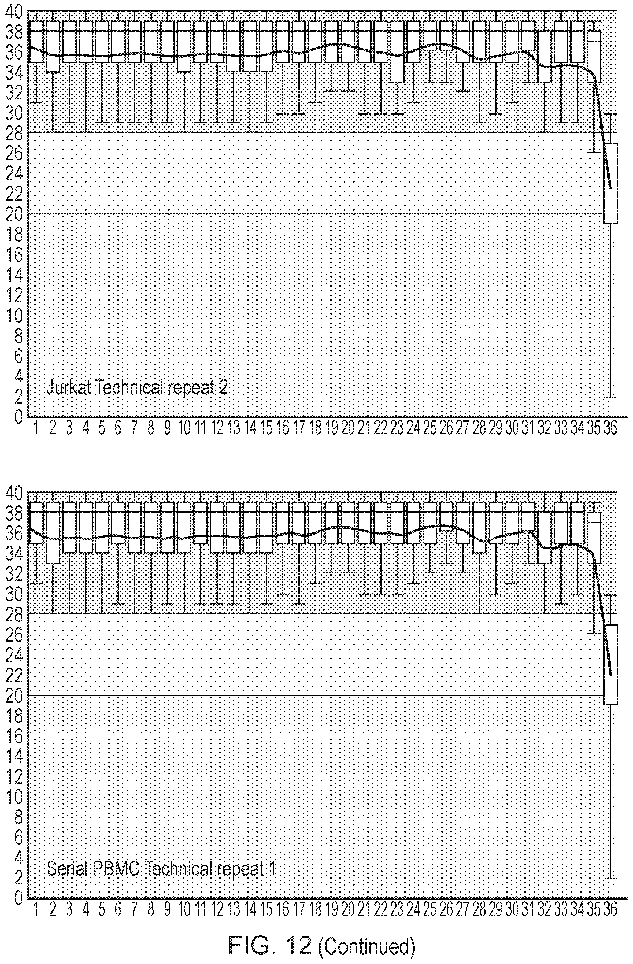

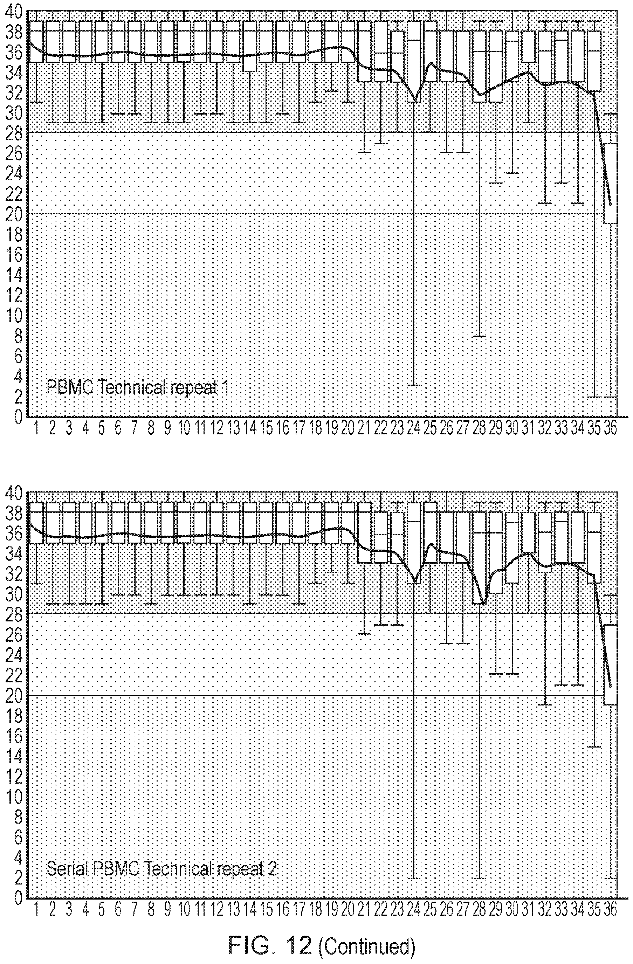

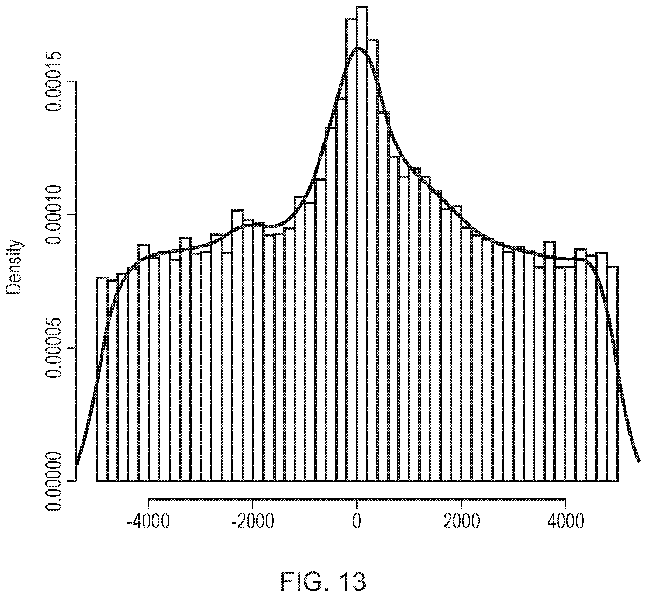

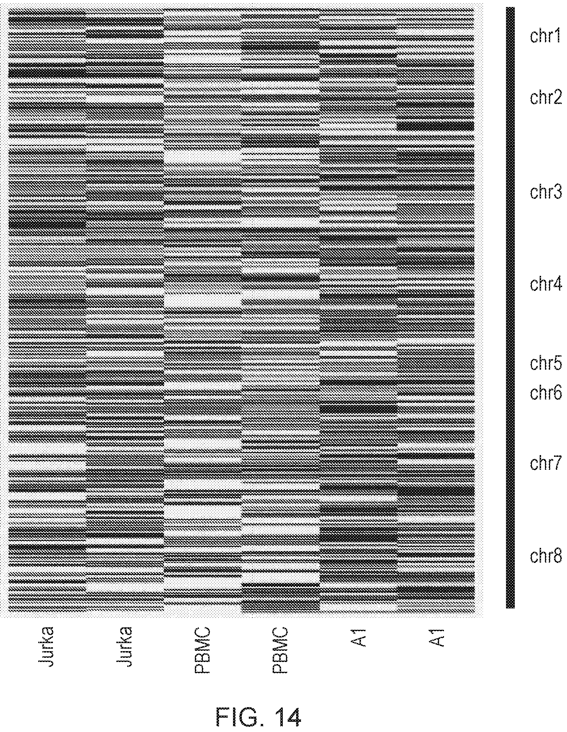

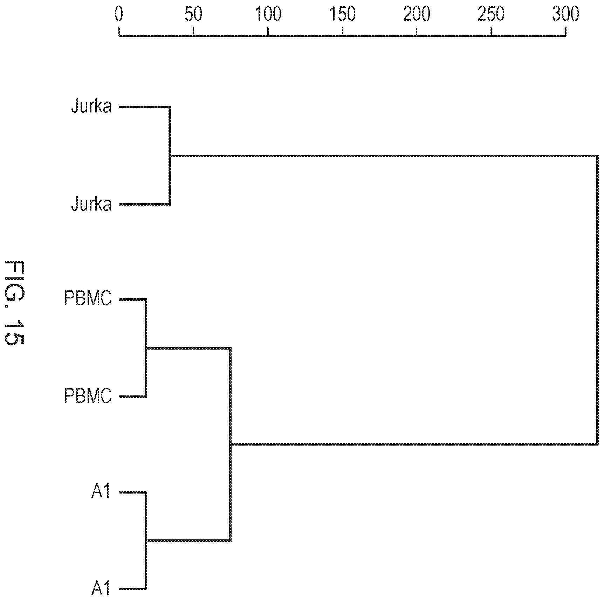

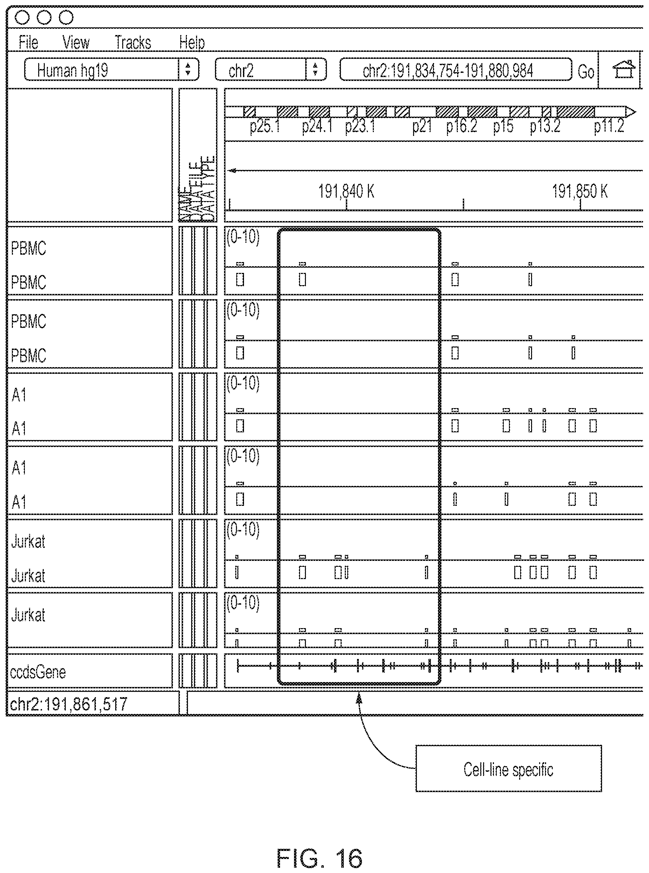

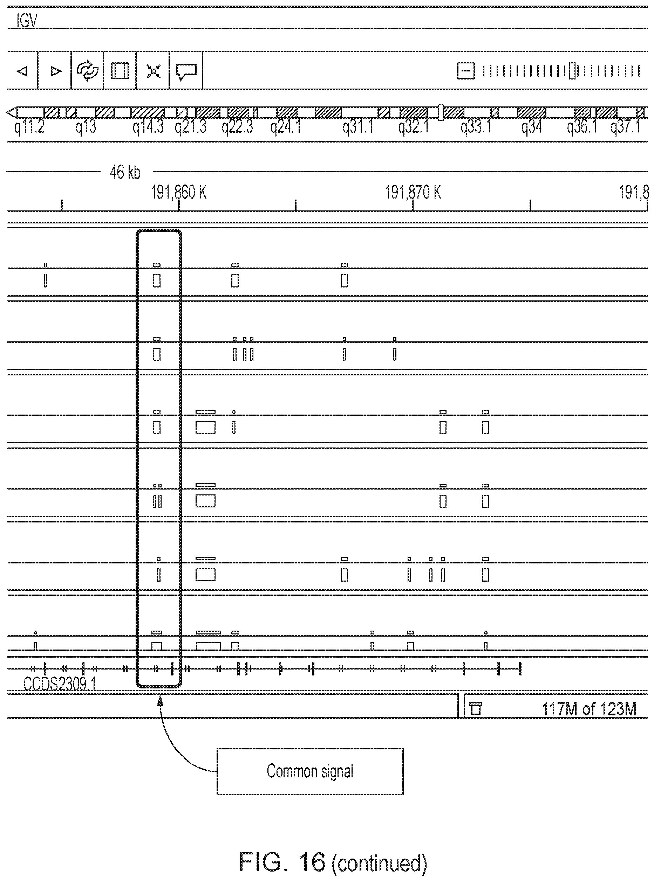

The read count per sample ranged from 30.1M-33.3M (normal range 28M-38M) with an almost perfect average base quality score of 38 (Figure 12). The reads were mapped to the reference human genome and were distributed across the genome with enrichment around transcription start sites, noted in the literature (see Collins et. al.; Genome Res; 2006; DOI 10.1101/gr.4074106) for their correlation with Nuclease Hypersensitivity (Figure 13). Importantly, the Nuclease Hypersensitive Site patterns identified using the bioinformatics pipeline shown in Figure 9 clearly grouped technical duplicates from each cell type and distinguished them from specific cell types as shown a heat plot of Nuclease Hypersensitive Sites across the first 8 chromosomes (Figure 14) and also shown as an rooted phylogenetic tree (Figure 15). An example of a common and a differential Nuclease Hypersensitive site is given in Figure 16, which is a view of a region of human chromosome 2 in a genome browser. The STAT1 gene is uniquely associated with Nuclease Hypersensitivity in Jurkat cells compared to the common signal located upstream which is seen in Jurkat cells as well as PBMCs and the first timed PBMC sample (A1).

**Example 5** Identification of differential Nuclease Hypersensitive sites in temporal samples of Peripheral Blood Mononuclear Cells (PBMCs) and demonstration of reproducibility between biological duplicates by rapid Genome wide screening

**Sequencing Data set B:** Four samples comprising Nuclease Hypersensitive site libraries from 1) Jurkat cells (3x10⁶ nuclei) digested with 0.07U/mL Nlalll for 10 minutes followed by on bead secondary digestion, ligation of secondary adapter and PCR amplification and 2) Jurkat cells (3x10⁶ nuclei) over-digested with 0.5U/mL Nlalll for 10 minutes followed by on bead secondary digestion, ligation of secondary adapter and PCR amplification were sequenced as singlets in separate lanes on an Illumina GAIIx platform (36 cycle, single read protocol), 3) The second and 4) third timed serial sample of PBMCs (3x10⁶ nuclei) digested with 0.07U/mL NIaIII for 10 minutes followed by on bead secondary digestion, ligation of secondary adapter and PCR amplification were sequenced on the remaining lanes as technical duplicates in separate lanes on an Illumina GAIIx platform (36 cycle, single read protocol).

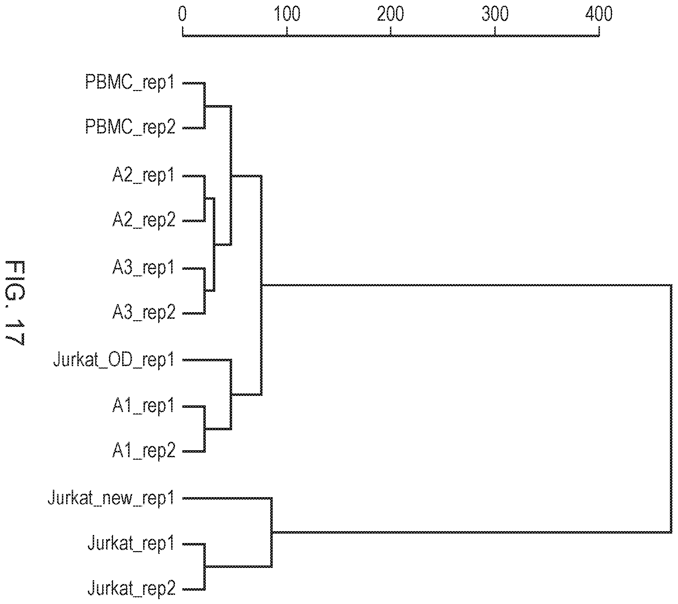

Clustering analysis of the combined A and B data sets showed good association of nuclease Hypersensitive sites in the second biological replicate of Jurkat new cells with the first set indicating that the method was reproducible as shown in the rooted phylogeny tree in Figure 17. A clear distinction between the Jurkat cells processed correctly and those that were over digested (Jurkat-OD) was noted. Also noteworthy is that the healthy PMBC cells clustered separately to the normally processed Jurkat cells indicated capability to differentiate healthy and diseased white blood cells. Finally the serially collected PBMC cells (A2-A3) also showed distinct clustering which associated more closely with healthy PBMCs and away from the first timed sample following a month of exercise and diet indicating capability to identify novel Nuclease Hypersensitive site profiles as potential biomarkers for fitness.

**Example 6** Identification of differential Nuclease Hypersensitive sites in Oestrogen stimulated and non-stimulated MCF7 cells by rapid Genome wide screening

**Sequencing Data set C:** Five samples comprising Nuclease Hypersensitive site libraries from 1) Jurkat cells (3x10⁶ nuclei) digested with 0.1U/mL Nlalll for 10 minutes followed by on bead secondary digestion, on bead ligation of secondary adapter and PCR amplification 2) Jurkat cells (3x10⁶ nuclei) overdigested with 0.5U/mL Nlalll for 10 minutes followed by off bead secondary digestion, ligation of secondary adapter and PCR amplification, 3) The second timed serial sample of PBMCs (3x10⁶ nuclei) digested with 0.07U/mL NIaIII for 10 minutes followed by on bead secondary digestion, on bead ligation of secondary adapter and PCR amplification were sequenced as singlicates on an Illumina HiSeq 2000 platform (36 cycle, single read protocol). MCF7 cells grown in culture without (4) and with (5) 10⁻⁷ M Oestrogen stimulation (3x10⁶ nuclei) digested with 0.07U/mL NIaIII for 10 minutes followed by on bead secondary digestion, ligation of secondary adapter and PCR amplification (as described in example 3) were sequenced in the remaining lanes of a HiSeq 2000 as technical duplicates.

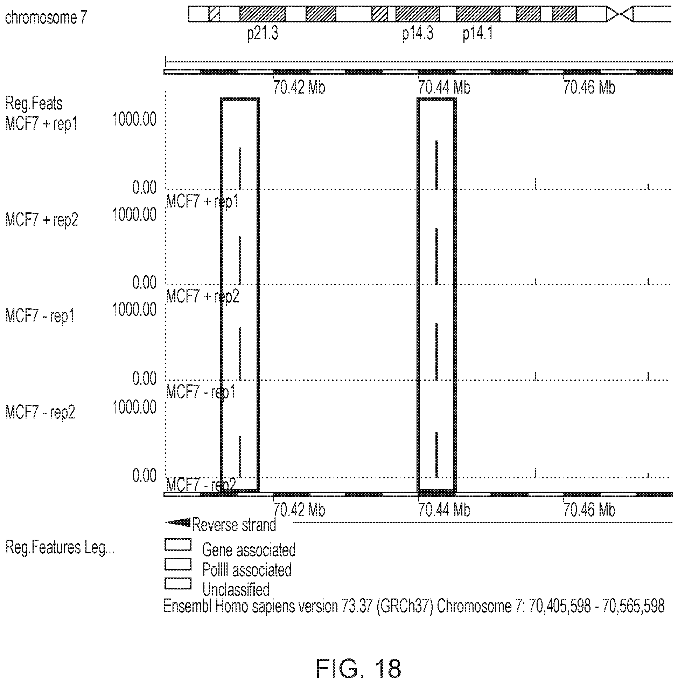

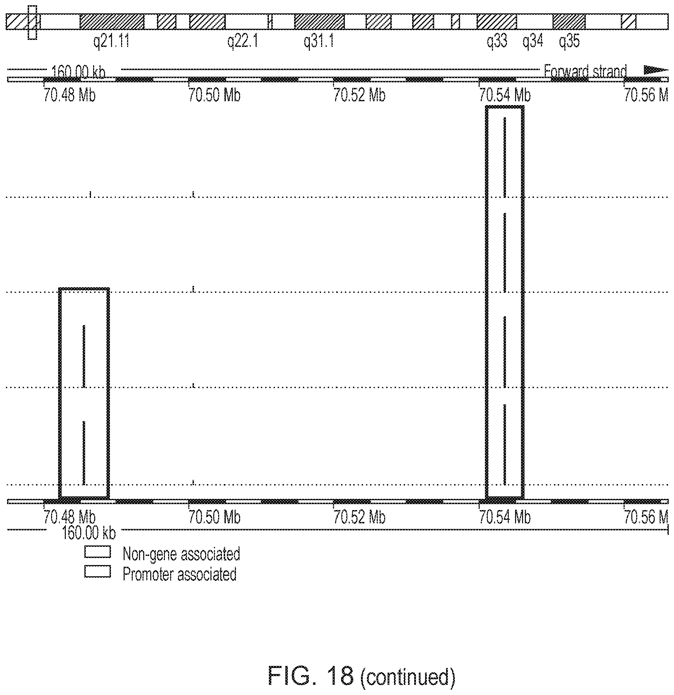

An example of three common Hypersensitive site in MCF 7 cells grown in the presence and absence of oestrogen as well as a differential Hypersensitive site present only in non oestrogen stimulated MCF7 cells within chromosome 7 is shown in Figure 18. Importantly the sites were identified outside of any genes in a region with few known regulatory elements.

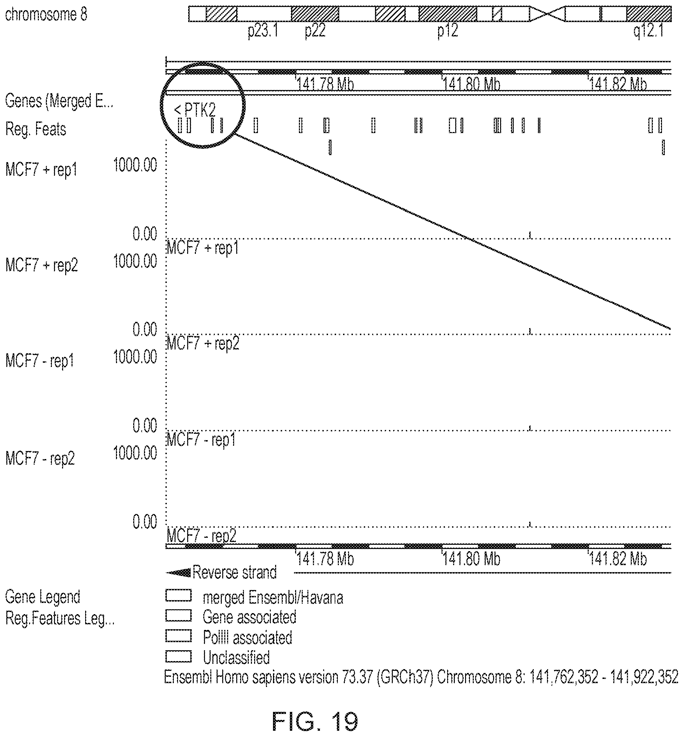

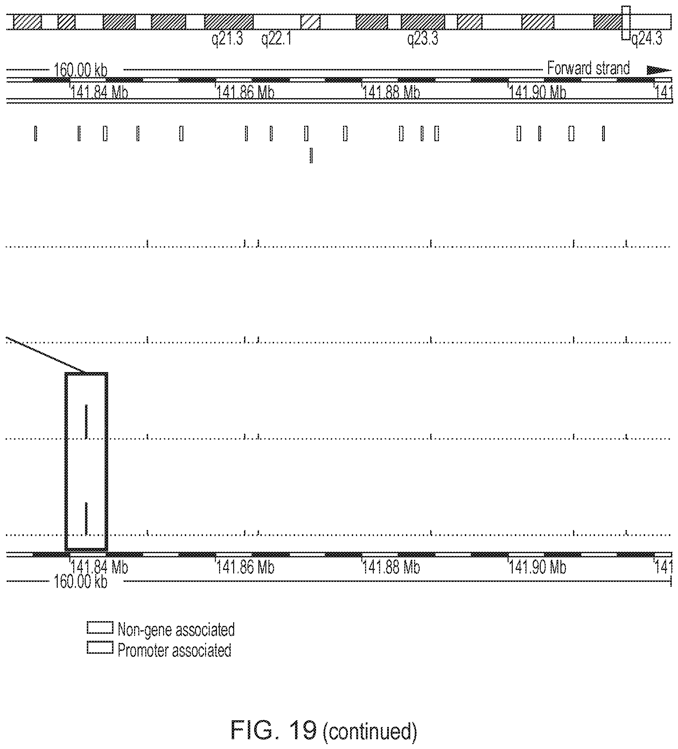

The identification of a differential Hypersensitive site within a known gene is illustrated in Figure 19 showing a Hypersensitive site within intron of the Protein Tyrosine Kinase 2 (PTK2) gene on chromosome 8 in non oestrogen stimulated MCF7 cells. The Hypersensitive site is not detected in oestrogen stimulated cells.

## Claims

1. A method for analysing nuclease hypersensitive sites which method comprises:
i) cleaving a nucleic acid sample comprising chromatin at multiple nuclease hypersensitive sites with a first sequence specific restriction enzyme to introduces a staggered cut and leave a single chain 3' or 5' overhang in a double stranded DNA;
ii) optionally isolating substantially free DNA from the digested nucleic acid sample or removing the protein and RNA components from the digested nucleic acid sample to leave substantially free DNA;
iii) ligating an adapter oligonucleotide onto the overhang produced by the first sequence specific restriction enzyme in aqueous solution, which adaptor oligonucleotide contains a single stranded region which is complementary to the overhang produced by the first sequence specific restriction enzyme, and which adaptor oligonucleotide contains a recognition site for a second restriction enzyme;
iv) treating the ligated DNA sequence with a second restriction enzyme wherein said second restriction enzyme is specific to said recognition site introduced within said adaptor oligonucleotide, wherein said second restriction enzyme cuts at a position at a defined number of bases distal to said recognition site and introduces a staggered cut, leaving a single chain 3' or 5' overhang in the double stranded DNA;
v) optionally amplifying the DNA fragments;
vi) analysing the DNA fragments formed in iv) or v) from a plurality of sequences (such as a plurality of genes)
wherein at least steps iii) and iv) of the method are conducted in an aqueous medium.

2. A method according to claim 1 wherein the method comprises after step (iv) a step of ligating a second oligonucleotide adaptor, which second oligonucleotide adaptor has a single stranded region which is complementary to the overhang produced by the second sequence specific restriction enzyme, to the DNA fragments.

3. A method according to claim 1 or claim 2 wherein:
a) the fragments are sequenced; and/or
b) the fragments are analysed on a hybridising array; and/or
c) the fragments are amplified by PCR.

4. A method according to claim 3 part c), wherein the first PCR primer hybridises to a sequence in the first adaptor and the second PCR primer hybridises to a sequence in the second adaptor or; wherein the first PCR primer hybridises to a sequence in the first adaptor and the second PCR primer hybridises to a known gene of interest.

5. A method according to any one of the preceding claims wherein the genomic DNA is obtained from a subject with a particular disease.

6. A method according to claim 5 wherein the method is repeated with genomic DNA obtained from a subject without said particular disease and the results thereof compared with the results from the genomic DNA obtained from a subject with a particular disease.

7. A method according to claim 6 comprising identifying differences between the diseased and non-diseased state.

8. A method according to claim 7 comprising identifying one or more biomarkers for a diseased state.

9. A method according to any one of claims 1-4 wherein the fragments (e.g. fragment patterns) from genomic DNA from a subject is compared with known fragments (e.g. fragment patterns) from genomic DNA which fragments (e.g. fragment patterns) have been associated with a disease, thus to determine whether the subject has said disease.

10. A method according to any one of the preceding claims comprising identifying one or more agents capable of modulating the fragments (e.g. fragment patterns) obtained from the genomic DNA of a subject.

11. A method according to any one of the preceding claims wherein the second sequence specific restriction enzyme cuts at a distance between 20 and 33 bp from its recognition site.

12. A method according to any one of the preceding claims wherein the second sequence specific restriction enzyme cuts at a distance of 22 bp from its recognition site; and/or
wherein the second sequence specific restriction enzyme is a Type IIG restriction enzyme, for example one selected from the group consisting of: Mmel TCCRAC(20/18), Acul CTGAAG(16/14), Bbsl GAAGAC(2/6), Bbvl GCAGC(8/12), BccI CCATC(4/5), BceAI ACGGC(12/14), BCiVI GTATCC(6/5), BcoDi GTCTC(1/5), BfuAI ACCTGC(4/8), BpuEi CTTGAG(16/14), BseRI GAGGAG(10/8), BsgI GTGCAG(16/14), BsmAI GTCTC(1/5), BSMBi CGTCTC(1/5), BSMFI GGGAC(10/14), BspCNI CTCAG(9/7), BSPQI GCTCTTC(1/4), EcoP15) CAGCAG(25/27), FokI GGATG(9/13), HgaI GACGC(5/10), HphI GGTGA(8/7), HpyAV CCTTC(6/5), MboII GAAGA(8/7), NmeAIII GCCGAG(21/19), and SapI GCTCTTC(1/4).

13. A method according to any one of the preceding claims wherein the aqueous medium comprises no or substantially no polymeric material (e.g. low melting point agarose) or other gelling agents; or the aqueous medium comprises less than 5g/L polymeric material (e.g. agarose) or other gelling agent.

14. A method according to any one of the preceding claims wherein the first sequence specific restriction enzyme is N1AIII, Fael or Hsp9211.

15. A kit for the preparation of hypersensitive site libraries which kit comprises:
i) a first sequence specific restriction enzyme capable of introducing a staggered cut and leaving a single chain 3' or 5' overhang in a double stranded DNA of a nucleic acid sample;
ii) an adapter oligonucleotide containing a single stranded region which is complementary to the overhang produced by the first sequence specific restriction enzyme, and which adaptor oligonucleotide contains a recognition site for a second restriction enzyme;
iii) a second restriction enzyme which is specific to said recognition site of said adaptor oligonucleotide, wherein said second restriction enzyme cuts at a position at a defined number of bases distal to said recognition site and introduces a staggered cut, leaving a single chain 3' or 5' overhang in the double stranded DNA.

## Patentansprüche

1. Verfahren zum Analysieren von Nukleasehypersensitiven Stellen, wobei das Verfahren umfasst:
i) Spalten einer Nukleinsäureprobe, umfassend Chromatin, an mehreren Nukleasehypersensitiven Stellen mit einem ersten sequenzspezifischen Restriktionsenzym, um einen versetzten Schnitt einzubringen und einen 3'- oder 5'-Einzelkettenüberhang in einer doppelsträngigen DNA zu hinterlassen;
ii) wahlweise Isolieren von im Wesentlichen freier DNA aus der verdauten Nukleinsäureprobe oder Entfernen der Protein- und RNA-Komponenten aus der verdauten Nukleinsäureprobe, um im Wesentlichen freie DNA zu hinterlassen;
iii) Ligieren eines Adapter-Oligonukleotids auf den Überhang, der durch das erste sequenzspezifische Restriktionsenzym in wässriger Lösung erzeugt wird, wobei das Adapter-Oligonukleotid einen einzelsträngigen Bereich enthält, der zu dem Überhang, der durch das erste sequenzspezifische Restriktionsenzym erzeugt wird, komplementär ist und wobei das Adapter-Oligonukleotid eine Erkennungsstelle für ein zweites Restriktionsenzym enthält;
iv) Behandeln der ligierten DNA-Sequenz mit einem zweiten Restriktionsenzym, wobei das zweite Restriktionsenzym spezifisch für die Erkennungsstelle ist, die innerhalb des Adapter-Oligonukleotids eingebracht wird, wobei das zweite Restriktionsenzym an einer Position mit einer definierten Zahl von Basen distal zu der Erkennungsstelle schneidet und einen versetzten Schnitt einbringt, wobei ein 3'- oder 5'-Einzelkettenüberhang in der doppelsträngigen DNA hinterlassen wird;
v) wahlweise Amplifizieren der DNA-Fragmente;
vi) Analysieren der DNA-Fragmente, die in iv) oder v) von mehreren Sequenzen (wie z.B. mehreren Genen) gebildet werden,
wobei mindestens Schritt iii) und iv) des Verfahrens in einem wässrigen Medium durchgeführt werden.

2. Verfahren nach Anspruch 1, wobei das Verfahren nach Schritt (iv) einen Schritt des Ligierens eines zweiten Oligonukleotid-Adapters an die DNA-Fragmente aufweist, wobei der zweite Oligonukleotid-Adapter einen einzelsträngigen Bereich aufweist, der zu dem Überhang, der von dem zweiten sequenzspezifischen Restriktionsenzym erzeugt wird, komplementär ist.

3. Verfahren nach Anspruch 1 oder 2, wobei:
a) die Fragmente sequenziert werden; und/oder
b) die Fragmente auf einem hybridisierenden Array analysiert werden; und/oder
c) die Fragmente mittels PCR amplifiziert werden.

4. Verfahren nach Anspruch 3, Teil c), wobei der erste PCR-Primer zu einer Sequenz in dem ersten Adapter hybridisiert und der zweite PCR-Primer zu einer Sequenz in dem zweiten Adapter hybridisiert; oder wobei der erste PCR-Primer zu einer Sequenz in dem ersten Adapter hybridisiert und der zweite PCR-Primer zu einem interessierenden bekannten Gen hybridisiert.

5. Verfahren nach einem der vorhergehenden Ansprüche, wobei die genomische DNA von einem Probanden mit einer bestimmten Krankheit gewonnen wird.

6. Verfahren nach Anspruch 5, wobei das Verfahren mit genomischer DNA wiederholt wird, die von einem Probanden ohne die bestimmte Krankheit gewonnen wird, und die Ergebnisse davon mit den Ergebnissen von der genomischen DNA verglichen werden, die von einem Probanden mit einer bestimmten Krankheit gewonnen wird.

7. Verfahren nach Anspruch 6, umfassend das Identifizieren von Unterschieden zwischen dem erkrankten und dem nicht erkrankten Zustand.

8. Verfahren nach Anspruch 7, umfassend das Identifizieren eines oder mehrerer Biomarker für einen erkrankten Zustand.

9. Verfahren nach einem von Anspruch 1 bis 4, wobei die Fragmente (z.B. Fragmentmuster) von genomischer DNA von einem Probanden mit bekannten Fragmenten (z.B. Fragmentmustern) von genomischer DNA verglichen werden, wobei die Fragmente (z.B. Fragmentmuster) einer Krankheit zugewiesen wurden, um so zu festzustellen, ob der Proband die Krankheit aufweist.

10. Verfahren nach einem der vorhergehenden Ansprüche, umfassend das Identifizieren eines oder mehrerer Mittel, die fähig sind, die Fragmente (z.B. Fragmentmuster), die von der genomischen DNA eines Probanden gewonnen werden, zu modulieren.

11. Verfahren nach einem der vorhergehenden Ansprüche, wobei das zweite sequenzspezifische Restriktionsenzym in einem Abstand von zwischen 20 und 33 bp von seiner Erkennungsstelle schneidet.

12. Verfahren nach einem der vorhergehenden Ansprüche, wobei das zweite sequenzspezifische Restriktionsenzym in einem Abstand von 22 bp von seiner Erkennungsstelle schneidet; und/oder wobei das zweite sequenzspezifische Restriktionsenzym ein Restriktionsenzym vom Typ IIG ist, beispielsweise eines, das aus der Gruppe ausgewählt wird, welche besteht aus: MmeI TCCRAC(20/18), AcuI CTGAAG(16/14), BbsI GAAGAC(2/6), BbvI GCAGC(8/12), BccI CCATC(4/5), BceAI ACGGC(12/14), BCiVI GTATCC(6/5), BcoDi GTCTC(1/5), BfuAI ACCTGC(4/8), BpuEi CTTGAG(16/14), BseRI GAGGAG(10/8), BsgI GTGCAG(16/14), BsmAI GTCTC(1/5), BSMBi CGTCTC(1/5), BSMFI GGGAC(10/14), BspCNI CTCAG(9/7), BSPQI GCTCTTC(1/4), EcoP15) CAGCAG(25/27), FokI GGATG(9/13), HgaI GACGC(5/10), HphI GGTGA(8/7), HpyAV CCTTC(6/5), MboII GAAGA (8/7), NmeAIII GCCGAG(21/19) und SapI GCTCTTC (1/4) .

13. Verfahren nach einem der vorhergehenden Ansprüche, wobei das wässrige Medium kein oder im Wesentlichen kein Polymermaterial (z.B. niedrigschmelzende Agarose) oder andere Geliermittel umfasst; oder
das wässrige Medium weniger als 5 g/l Polymermaterial (z.B. Agarose) oder andere Geliermittel umfasst.

14. Verfahren nach einem der vorhergehenden Ansprüche, wobei das erste sequenzspezifische Restriktionsenzym N1AIII, FaeI oder Hsp9211 ist.

15. Kit zur Herstellung von Bibliotheken von hypersensitiven Stellen, wobei das Kit umfasst:
i) ein erstes sequenzspezifisches Restriktionsenzym, das fähig ist, einen versetzten Schnitt einzubringen und einen 3'- oder 5'-Einzelkettenüberhang in einer doppelsträngigen DNA einer Nukleinsäureprobe zu hinterlassen;
ii) ein Adapter-Oligonukleotid, das einen einzelsträngigen Bereich enthält, der zu dem Überhang, der durch das erste sequenzspezifische Restriktionsenzym erzeugt wird, komplementär ist und wobei das Adapter-Oligonukleotid eine Erkennungsstelle für ein zweites Restriktionsenzym enthält;
iii) ein zweites Restriktionsenzym, das für die Erkennungsstelle des Adapter-Oligonukleotids spezifisch ist, wobei das zweite Restriktionsenzym an einer Position mit einer definierten Zahl von Basen distal zu der Erkennungsstelle schneidet und einen versetzten Schnitt einbringt, wobei ein 3'- oder 5'-Einzelkettenüberhang in der doppelsträngigen DNA hinterlassen wird.

## Revendications

1. Procédé d'analyse de sites hypersensibles aux nucléases, ledit procédé comprenant :
i) le clivage d'un échantillon d'acide nucléique comprenant de la chromatine à des sites hypersensibles aux nucléases multiples avec une première enzyme de restriction spécifique à une séquence pour introduire une coupure décalée et laisser une extrémité saillante en 3' ou 5' monocaténaire dans un ADN double brin ;
ii) facultativement, l'isolement d'ADN sensiblement libre à partir de l'échantillon d'acide nucléique digéré ou la séparation des composants de protéine et d'ARN à partir de l'échantillon d'acide nucléique digéré pour laisser de l'ADN sensiblement libre ;
iii) la ligature d'un oligonucléotide adaptateur sur l'extrémité saillante produite par la première enzyme de restriction spécifique à une séquence en solution aqueuse, ledit oligonucléotide adaptateur contenant une région simple brin qui est complémentaire de l'extrémité saillante produite par la première enzyme de restriction spécifique à une séquence, et ledit oligonucléotide adaptateur contenant un site de reconnaissance pour une deuxième enzyme de restriction ;
iv) le traitement de la séquence d'ADN ligaturée avec une deuxième enzyme de restriction, ladite deuxième enzyme de restriction étant spécifique pour ledit site de reconnaissance introduit dans ledit oligonucléotide adaptateur, dans lequel ladite deuxième enzyme de restriction coupe à une position à un nombre défini de bases distales par rapport audit site de reconnaissance et introduit une coupure décalée, laissant une extrémité saillante en 3' ou 5' monocaténaire dans l'ADN double brin ;
v) facultativement, l'amplification des fragments d'ADN ;
vi) l'analyse des fragments d'ADN formés dans iv) ou v) à partir d'une pluralité de séquences (telle qu'une pluralité de gènes)
dans laquelle au moins les étapes iii) et iv) du procédé sont conduites dans un milieu aqueux.

2. Procédé selon la revendication 1, le procédé comprenant, après l'étape (iv), une étape de ligature d'un deuxième oligonucléotide adaptateur, ledit deuxième oligonucléotide adaptateur comportant une région simple brin qui est complémentaire de l'extrémité saillante produite par la deuxième enzyme de restriction spécifique à une séquence, aux fragments d'ADN.

3. Procédé selon la revendication 1 ou la revendication 2 dans lequel :
a) les fragments sont séquencés ; et/ou
b) les fragments sont analysés sur une puce d'hybridation ; et/ou
c) les fragments sont amplifiés par PCR.

4. Procédé selon la revendication 3 partie c), dans lequel la première amorce de PCR s'hybride à une séquence dans le premier adaptateur et la deuxième amorce de PCR s'hybride à une séquence dans le deuxième adaptateur ou ;
dans lequel la première amorce de PCR s'hybride à une séquence dans le premier adaptateur et la deuxième amorce de PCR s'hybride à un gène d'intérêt connu.

5. Procédé selon l'une quelconque des revendications précédentes, dans lequel l'ADN génomique est obtenu à partir d'un sujet ayant une maladie particulière.

6. Procédé selon la revendication 5, le procédé étant répété avec de l'ADN génomique obtenu à partir d'un sujet sans ladite maladie particulière et les résultats de celui-ci étant comparés aux résultats de l'ADN génomique obtenus à partir d'un sujet ayant une maladie particulière.

7. Procédé selon la revendication 6, comprenant l'identification de différences entre l'état malade et non malade.

8. Procédé selon la revendication 7, comprenant l'identification d'un ou plusieurs biomarqueurs pour un état malade.

9. Procédé selon l'une quelconque des revendications 1 à 4, dans lequel les fragments (par ex. des motifs de fragment) d'ADN génomique d'un sujet sont comparés à des fragments connus (par ex. des motifs de fragment) d'ADN génomique, lesdits fragments (par ex. des motifs de fragment) ayant été associés à une maladie, de façon à déterminer si le sujet présente ladite maladie.

10. Procédé selon l'une quelconque des revendications précédentes comprenant l'identification d'un ou plusieurs agents capables de moduler les fragments (par ex. des motifs de fragment) obtenus à partir de l'ADN génomique d'un sujet.

11. Procédé selon l'une quelconque des revendications précédentes dans lequel la deuxième enzyme de restriction spécifique à une séquence coupe à une distance comprise entre 20 et 33 pb de son site de reconnaissance.

12. Procédé selon l'une quelconque des revendications précédentes dans lequel la deuxième enzyme de restriction spécifique à une séquence coupe à une distance de 22 pb de son site de reconnaissance ; et/ou
dans lequel la deuxième enzyme de restriction spécifique à une séquence est une enzyme de restriction de type IIG, par exemple l'une choisie dans le groupe constituée de : MmeI TCCRAC(20/18), AcuI CTGAAG(16/14), BbsI GAAGAC(2/6), BbvI GCAGC(8/12), BccI CCATC(4/5), BceAI ACGGC(12/14), BCiVI GTATCC(6/5), BcoDi GTCTC(1/5), BfuAI ACCTGC(4/8), BpuEi CTTGAG(16/14), BseRI GAGGAG(10/8), BsgI GTGCAG(16/14), BsmAI GTCTC(1/5), BSMBi CGTCTC(1/5), BSMFI GGGAC(10/14), BspCNI CTCAG(9/7), BSPQI GCTCTTC(1/4), EcoP15) CAGCAG(25/27), FokI GGATG(9/13), HgaI GACGC(5/10), HphI GGTGA(8/7), HpyAV CCTTC(6/5), MboII GAAGA(8/7), NmeAIII GCCGAG(21/19) et SapI GCTCTTC(1/4).

13. Procédé selon l'une quelconque des revendications précédentes dans lequel le milieu aqueux ne comprend aucun ou sensiblement aucun matériau polymère (par ex. de l'agarose à bas point de fusion) ou d'autres agents gélifiants ; ou
le milieu aqueux comprend moins de 5 g/l de matériau polymère (par ex. l'agarose) ou un autre agent gélifiant.

14. Procédé selon l'une quelconque des revendications précédentes dans lequel la première enzyme de restriction spécifique à une séquence est N1AIII, FaeI ou Hsp9211.

15. Kit de préparation de banques de sites hypersensibles, ledit kit comprenant :
i) une première enzyme de restriction spécifique à une séquence capable d'introduire une coupure décalée et de laisser une extrémité saillante en 3' ou 5' monocaténaire dans un ADN double brin d'un échantillon d'acide nucléique ;
ii) un oligonucléotide adaptateur contenant une région simple brin qui est complémentaire de l'extrémité saillante produite par la première enzyme de restriction spécifique à une séquence, et ledit oligonucléotide adaptateur contenant un site de reconnaissance pour une deuxième enzyme de restriction ;
iii) une deuxième enzyme de restriction qui est spécifique audit site de reconnaissance dudit oligonucléotide adaptateur, dans lequel ladite deuxième enzyme de restriction coupe à une position à un nombre défini de bases distales par rapport audit site de reconnaissance et introduit une coupure décalée, laissant une extrémité saillante en 3' ou 5' monocaténaire dans l'ADN double brin.


### SEQUENCE LISTING

<110> HyperGenomics PTE. Limited
<120> METHOD
<130> P100411PCT
<150> GB 1506315.9
   <151> 2015-04-14
<160> 9
<170> PatentIn version 3.5
<210> 1
   <211> 26
   <212> DNA
   <213> Artificial Sequence
<220>
   <223> Target sequence of MmeI
<220>
   <221> misc_feature
   <222> (7)..(26)
   <223> n is a, c, g, or t
<400> 1
   tccracnnnn nnnnnnnnnn nnnnnn 26
<210> 2
   <211> 24
   <212> DNA
   <213> Artificial Sequence
<220>
   <223> Target sequence of MmeI
<220>
   <221> misc_feature
   <222> (1)..(18)
   <223> n is a, c, g, or t
<400> 2
   nnnnnnnnnn nnnnnnnngt ygga 24
<210> 3
   <211> 29
   <212> DNA
   <213> Artificial Sequence
<220>
   <223> Illumina NIaIII gene expression oligonucleotide sequence
<400> 3
   acaggttcag agttctacag tccgacatg 29
<210> 4
   <211> 21
   <212> DNA
   <213> Artificial Sequence
<220>
   <223> Illumina NIaIII gene expression oligonucleotide sequence
<400> 4
   tcggactgta gaactctgaa c 21
<210> 5
   <211> 22
   <212> DNA
   <213> Artificial Sequence
<220>
   <223> Oligonucleotide sequence for primary adapter
<400> 5
   gtcggactgt agaactctga ac 22
<210> 6
   <211> 23
   <212> DNA
   <213> Artificial Sequence
<220>
   <223> Oligonucleotide sequence for adapter 2
<220>
   <221> misc_feature
   <222> (22)..(23)
   <223> n is a, c, g, or t
<400> 6
   caagcagaag acggcatacg ann 23
<210> 7
   <211> 21
   <212> DNA
   <213> Artificial Sequence
<220>
   <223> Oligonucleotide sequence for adapter 2
<400> 7
   tcgtatgccg tcttctgctt g 21
<210> 8
   <211> 21
   <212> DNA
   <213> Artificial Sequence
<220>
   <223> PCR primer 1
<400> 8
   caagcagaag acggcatacg a 21
<210> 9
   <211> 44
   <212> DNA
   <213> Artificial Sequence
<220>
   <223> PCR primer 2
<400> 9
   aatgatacgg cgaccaccga caggttcaga gttctacagt ccga 44